# EUROPEAN PATENT SPECIFICATION

(11) **EP 4 090 286 B1**
(45) Date of publication and mention of the grant of the patent: **10.12.2025**
(21) Application number: 21741201.4
(22) Date of filing: 11.01.2021
(51) Int. Cl.: A61F 2/16

(54) **INTRAOCULAR RING SYSTEM**
INTRAOKULARES RINGSYSTEM
SYSTÈME D'ANNEAU INTRAOCULAIRE

(30) Priority: 13.01.2020 US 202062960156 P
(43) Date of publication of application: 23.11.2022
(73) Proprietor: Shaare Zedek Scientific Ltd., 9103102 Jerusalem (IL); Wertzberger, Shay Roy, 6249411 Tel Aviv (IL); Howard, Adam Kenneth, 4535957 Hod HaSharon (IL)
(72) Inventor: WERTZBERGER, Shay Roy, 6249411 Tel Aviv (IL); ABULAFIA, Adi, 6291056 Tel Aviv (IL); HOWARD, Adam Kenneth, 4535957 Hod HaSharon (IL)
(74) Representative: Caldon, Giuliano
(86) International application number: PCT/IL2021/050031
(87) International publication number: WO 2021/144787

(56) References cited:
- WO-A1-2018/229766
- WO-A1-2018/229766
- WO-A2-2015/198236
- US-A- 5 108 429
- US-A- 5 108 429
- US-A- 5 800 533
- US-A1- 2006 089 712
- US-A1- 2007 050 024
- US-A1- 2007 260 308
- US-A1- 2008 306 588
- US-A1- 2010 016 963
- US-A1- 2012 323 320
- US-A1- 2015 327 991
- US-B2- 7 316 713
- US-B2- 9 795 473

## Description

### FIELD OF THE INVENTION

The present invention generally pertains to a system for providing an adjustable intraocular ring system.

### BACKGROUND OF THE INVENTION

There has long been a need for a system to enable the implantation of ocular lenses which allows for the adjustment of the ocular lens post implantation in an easy and non-invasive manner. Several attempts have been made to develop systems to meet this need.

U.S. granted patent no. US8,728,158 to Jeffrey C. Whitsett is for an exchangeable intraocular lens device having a flexible, high-memory expansile lens fixation platform adapted to receive the haptics of an intraocular lens, and their method of use, are provided. The device is specifically designed to expand completely into the equatorial fornix of the capsular bag and become permanently implanted therein. US8,728,158 addresses the desire of patients to exchange their existing intraocular lens to meet their changing visual needs or to take advantage of improved lens technology, without incurring the significant risks typically associated with exchange of current intraocular lens technology. The exchangeable intraocular lens device provides accurate centration, positioning, and stability of the intraocular lens in the capsular bag. The exchangeable intraocular lens device reduces lens epithelial cell migration and resultant posterior capsule opacification.

However, it requires the use of a lens of a design unique to the patent that allows only a limited amount of angular adjustment and the lens must be rotated manually, thus requiring at least minimally invasive rotation of the lens.

U.S. granted patent no. US8,998,984 to Andrew F. Phillips is for an adjustable astigmatism-reducing intraocular lens including a toric optic with a long axis. The optic is rotationally coupled to haptics, and a plurality of struts extend between the optic and the haptics. The struts are held under tension and individually releasable via laser or are heat shrinkable to increase the tension of selective struts. When a strut is released or shrunk, a torsional force is applied to angularly adjust the optic relative to the haptics. After the lens has been implanted and healed relative to the tissue, struts are individually released via laser ablation to cause torsional instability and resulting net rotational adjustment, or individually heat shrunk to tension the strut, to cause torsional instability, and effect net rotational adjustment to ensure that a long axis of optic is aligned with the axis of astigmatic correction of the eye

However, the device of US8,998,984 allows angular rotations only by fixed amounts. Furthermore, since the struts which generate the angular adjustment are damaged or destroyed in order to generate the adjustment, only a limited number of adjustments can be made and are irreversible (e.g., a heat-shrunk strut can be shrunk or broken but cannot be expanded). In addition, although the lens is removable (by breaking the struts) the function of the toric optics of US8,998,984 cannot be replaced as there does not appear to be any means by which a new strut can be attached to a haptic in place in the eye.

European patent EP3638153 teaches a lens support structure for supporting an intraocular lens (IOL). The lens support structure is configured to be securely implanted in a lens capsule of a human eye and to hold the IOL in one of a plurality of positions, the support structure comprising a repositioning assembly configured to be activated remotely by a remote energy source and controllably displace the IOL in at least one of directions along and around an optical axis of the IOL, thereby enabling moving the IOL between the plurality of positions. A lens control system is also provided, the control system comprising the lens support structure and a source energy for activating parts thereof. An intraocular lens system is also provided, the system comprising the lens support structure and a lens integrated therein.

However, the IOL of EP3638153 requires a proprietary IOL, with intermediate ridges to connect it to the adjustment rings. Furthermore, the lens is permanently mounted in the innermost intermediate ring and can not move with respect to the innermost intermediate ring.

WIPO Patent Application Publication No. WO2018/229766 teaches a lens support structure for supporting an intraocular lens (IOL), the lens support structure being configured and operable to be securely implanted in a lens capsule of a human eye and hold the IOL in one of a plurality of positions, the support structure comprising a repositioning assembly configured and operable to be activated remotely by a remote energy source and controllably displace the IOL in at least one of directions along and around an optical axis of the IOL, thereby enabling moving the IOL between the plurality of positions. Lens control system is also provided, the control system comprising the lens support structure and a source energy for activating parts thereof. Intraocular lens system is also provided, the system comprising the lens support structure and a lens integrated therein.

However, the IOL of WO2018/229766 uses heating of a shape-memory alloy to move the optic relative to the lens support structure, and is not capable of tilt, decentering or angulation.

U.S granted patent US5108429 teaches an adjustable focus lens apparatus that includes a transparent lens body having a periphery, an attachment device adjacent to the periphery of the lens body for mounting the lens apparatus in an eye, and a plurality of micromotor devices spaced equally about and connected between the periphery of the lens body and the attachment means, each of the micromotor devices being responsive to an external control signal for selectively changing the position of an associated portion of the lens body with respect to the cornea and retina so that the functional power and astigmatism of the lens can be appropriately adjusted.

However, the apparatus of US5108429, requires a micromotor internal to the eye, and is not capable of tilt, decentering or angulation.

It is therefore a long felt need to provide an adjustable intraocular ring system that does not require use of a lens unique to the system and whose adjustment is non-invasive, reversible and highly calibrated to an individual patient's need.

### SUMMARY OF THE INVENTION

The present invention relates to an intraocular ring system (IOL) as set forth in the appended claims.

The method of using the system does not form part of the invention.

In particular the system comprises:
a primary intraocular ring (PIR) mountable to an eye; and
a secondary device (SD), said SD communicable with said PIR and movable relative to said PIR, said communication selected from a group consisting of rotatable, forward-backward movable, decentralizable, tiltable, angulatable and any combination thereof,
wherein said IOL has controllable and non-invasive movement relative to said PIR, said movement selected from a group consisting of rotation, forward-backward movement, decentralization, tilt, angulation and any combination thereof.

It is another object of the present invention to disclose the support system for an IOL as described above, wherein said IOL has at least one optic, said at least one optic having at least one haptic. It is another object of the present invention to disclose the support system for an IOL as described above, wherein at least one of said at least one haptic is flexible.

It is another object of the present invention to disclose the support system for an IOL as described above, wherein each of said at least one haptic is either a plate haptic or a loop haptic.

It is another object of the present invention to disclose the support system for an IOL as described above, wherein said IOL is a conventional IOL.

It is another object of the present invention to disclose the support system for an IOL as described above, wherein said PIR is configured to be supported by a member of a group consisting of the zonula of the eye, the capsular bag of the eye and any combination thereof.

It is another object of the present invention to disclose the support system for an IOL as described above, wherein at least one of the following is true:
a. said SD is removably attachable to said PIR;
b. said PIR is connectable to said SD by means of a holding structure comprising a male part and a female part, said male part mating with said female part, said male part configured by means of size and shape to fit into said female part snugly and movably, a shape of a cross section of said holding structure selected from a group consisting of a tongue and groove, a U shape, a C shape, a V shape, a truncated V shape, a zigzag, and any combination thereof;
c. said PIR is connectable to said SD by means of a holding structure comprising a male part and a female part, said male part mating with said female part, said male part configured by means of size and shape to fit into said female part snugly and movably, said connection between said male part and said female part selected from a group consisting of intercalated, overlapping, interlocking and any combination thereof;
d. an outward side of said SD is contactable to an inward side of said PIR, said outward side of said SD having a cross-section shape selected from a group consisting of rectangular, oval, circular, elliptical, polygonal and any combination thereof; said inward side of said PIR having a cross-section shape selected from a group consisting of rectangular, oval, circular, elliptical, polygonal and any combination thereof;
e. said SD has a shape selected from a group consisting of circular, oval, elliptical, segmented, and any combination thereof;
f. said SD comprises a member of a group consisting of a spring attachable to said PIR, an elastic band attachable to said PIR, a tab, a bar and any combination thereof;
g. said PIR comprises flexible material, rigid material and any combination thereof;
h. said SD comprises flexible material, rigid material and any combination thereof;
i. said PIR is a partial ring, comprising a gap;
j. said SD is a partial ring, comprising at least one gap;
k. a member of a group consisting of said PIR, said SD and any combination thereof additionally comprises at least one interstice;
l. said SD is either connected to said IOL without connection to said PIR or said SD is attachable to said IOL and attachable to said PIR; and
m. said attachment of said IOL to said SD is either a permanent attachment or a removable attachment.

It is another object of the present invention to disclose the support system for an IOL as described above, wherein a member of a ring set is selected from a group consisting of said IOL, said SD and any combination thereof.

It is another object of the present invention to disclose the support system for an IOL as described above, wherein a mechanism of said movement of a member of said ring set relative to said PIR is selected from a group consisting of a motor, a spring, a magnetic material, a magnet, an electromagnet, an ultrasonic field, a screw-like geometry of the PIR, a screw-like geometry of the SD, a shape memory alloy (SMA), a thermal actuator, an electro-reactive actuator, a shape shift material actuator, a heat reactive material, an elastic material, a screw, and any combination thereof.

It is another object of the present invention to disclose the support system for an IOL as described above, wherein at least one of the following is true:
a. power for said motor is providable by a means selected from a group consisting of a battery, a capacitor, an induction ring, laser light, ultrasound energy and any combination thereof;
b. power for said electromagnet is providable by a means selected from a group consisting of a battery, a capacitor, an induction ring, laser light, ultrasound energy and any combination thereof; and
c. said motor is selected from a group consisting of an electric motor, a piezoelectric motor, a sonic motor, a twisted polymer and any combination thereof.

It is another object of the present invention to disclose the support system for an IOL as described above, wherein a member of a group consisting of said PIR, said SD and any combination thereof has a back.

It is another object of the present invention to disclose the support system for an IOL as described above, wherein said back is selected from a group consisting of a solid back, a non-solid back and a partial back.

It is another object of the present invention to disclose the support system for an IOL as described above, wherein a non-solidity of said back is selected from a group consisting of a perforation, a slot, a hole, a regularly shaped gap, an irregularly shaped gap, a mesh, and any combination thereof.

It is another object of the present invention to disclose the support system for an IOL as described above, wherein said back comprises transparent material.

It is another object of the present invention to disclose the support system for an IOL as described above, wherein said PIR has at least one IOL guide configured by means of size and shape to guide said at least one haptic into a correct location.

It is another object of the present invention to disclose the support system for an IOL as described above, wherein said SD has at least one IOL guide configured by means of size and shape to guide said at least one haptic into a correct location.

It is another object of the present invention to disclose the support system for an IOL as described above, wherein said SD comprises at least one component support configured by means of size and shape to hold at least a portion of a component selected from a group consisting of an IOL, an SD and any combination thereof.

It is another object of the present invention to disclose the support system for an IOL as described above, wherein said at least one component support is selected from a group consisting of a mounting groove, a V groove, a U, an indentation and any combination thereof.

It is another object of the present invention to disclose the support system for an IOL as described above, wherein at least a portion of said at least one haptic is held snugly by said at least one component support.

It is another object of the present invention to disclose the support system for an IOL as described above, wherein said SD comprises a plurality of component supports, each of said plurality of component supports configured by means of size and shape to hold at least one IOL.

It is another object of the present invention to disclose the support system for an IOL as described above, wherein said PIR comprises said plurality of component supports, each of said plurality of component supports configured by means of size and shape to hold an SD.

In order to better understand the invention and its implementation in practice, a plurality of embodiments will now be described, by way of non-limiting example only, with reference to the accompanying drawings, wherein
Fig. 1A-D illustrates the prior art;
Fig. 2 shows a coordinate system for the eye;
Fig. 3A-H and Fig. 4A-L show examples of IOLs;
Fig. 5 illustrates an embodiment of the present invention;
Fig. 6 illustrates an embodiment of the primary intraocular ring and secondary device;
Figs. 7A-B, 8, 9 and 10A-B illustrate embodiments of the present invention;
Fig. 11A-C schematically illustrates a cross-section of an embodiment of the primary intraocular ring and secondary device;
Fig. 12 illustrates an embodiment of the present invention which comprises indentations to allow stable positioning of the IOL in the IRS;
Fig. 13A-C illustrates an embodiment of the present invention which comprises a ratchet mechanism to allow stable positioning of the IOL in the IRS;
Fig. 14 illustrates an embodiment of the present invention which comprises tabs to accept ultrasonic waves for rotation of the SD relative to the PIR;
Fig. 15A-B illustrates an embodiment of the present invention which comprises tabs to accept ultrasonic waves for rotation and translation of the SD relative to the PIR;
Fig. 16 illustrates an embodiment of the present invention in which the secondary device is not connected to the primary intraocular ring;
Figs. 17-22 illustrate embodiments of the present invention;
Fig. 23A-C illustrates embodiments of the IRS with two or more SDs;
Fig. 23D illustrates an embodiment of an SD without a male-female connection to an adjoining SD;
Fig. 24A-B schematically illustrates cross-sections of an embodiment of the primary intraocular ring and secondary devices; and
Fig. 25A-B schematically illustrate embodiments of a PIR with interstices.

### DETAILED DESCRIPTION OF THE PREFERRED EMBODIMENTS

The following description is provided, alongside all chapters of the present invention, so as to enable any person skilled in the art to make use of said invention and sets forth the best modes contemplated by the inventor of carrying out this invention. Various modifications, however, will remain apparent to those skilled in the art, since the generic principles of the present invention have been defined specifically to provide a means and method for providing an adjustable intraocular ring system.

The term **"intraocular lens" or "IOL"** hereinafter refers to an artificial lens implantable in an eye. An IOL comprises at least one refractive portion, typically approximately circular and typically approximately in the center of the IOL, and at least one haptic.

The term **"optic"** hereinafter refers to the refractive portion of an IOL.

The term **"haptic"** hereinafter refers a portion of an IOL which extends away from the optic. The haptic(s) are configured to hold the IOL in position relative to another object, such as an eye, with or without subsidiary holding structures.

The term **"conventional"** hereinafter refers to an item commonly or commercially available and, typically, configured for use with a plurality of mounting systems or no mounting system. An IOL comprising a central optic and two J haptics is a non-limiting example of a conventional IOL. In contrast, a "proprietary IOL" is an IOL configured for use with only one mounting system. Typically, the IOL and the mounting system are only available from one supplier and, typically, both are available from the same supplier.

In a normal eye, the lens is clear and the cornea has smooth front and back surfaces, each surface approximating a cap of a sphere. However, if the lens becomes cloudy (cataract), the progressively increasing cloudiness will affect vision. A non-smooth front or back corneal surface or a corneal surface which does not approximate a spherical surface can also affect vision independently of lens clarity, resulting in astigmatism. The treatment, for cataracts when vision is sufficiently affected, is to remove the affected lens and, preferably, replace it with an artificial lens. In order to provide satisfactorily clear and sharp vision after surgery, the artificial lens needs to match characteristics of the eye into which it is implanted. The most important of these characteristics are the refractive power of the eye, the astigmatism of the eye and the axis of the astigmatism.

The diopter describes the focusing power of a lens. In order to have clear vision, light needs to be focused onto the fovea; for light coming from infinity, a focal point in front of the fovea is typical of shortsightedness, while a focal point behind the fovea is typical of farsightedness. In an implanted artificial lens, the variables are diopter and the effective lens position in the eye; the combination should be matched to the eye for clear vision at any desired distance.

Cylinder describes the correction for corneal astigmatism, the non-smoothness and non-sphericalness of the cornea. An artificial lens can be designed with a semi-cylindrical shape (a toric lens); the cylindrical axis of a toric IOL can then be matched to the astigmatic meridian of the cornea to reduce or eliminate post-operative astigmatic refraction. The cylindrical axes of the toric IOL and the cornea must be matched; for every degree of misalignment from the desired axis, there is a 3% loss of cylinder correction. Therefore, the lens must be rotationally stable in the eye, or its cylinder axis must be adjustable. In practice, most implantations do not successfully achieve an optimal toric alignment, non-limiting examples for this include: incomplete alignment by the surgeon during implantation, rotation of the IOL post-implantation, miscalculation of the toric power axis by medical personnel or miscalculation of the toric power axis during lens manufacture or lens selection. Additionally, after a lens has been implanted, unpredictable rotational shifts can occur during post-operative healing of the capsular remnant, which can result in the lens rotating relative to the initial axis of implantation. As a result, most IOL recipients do not achieve the clear vision at the desired distance that was defined pre-implantation.

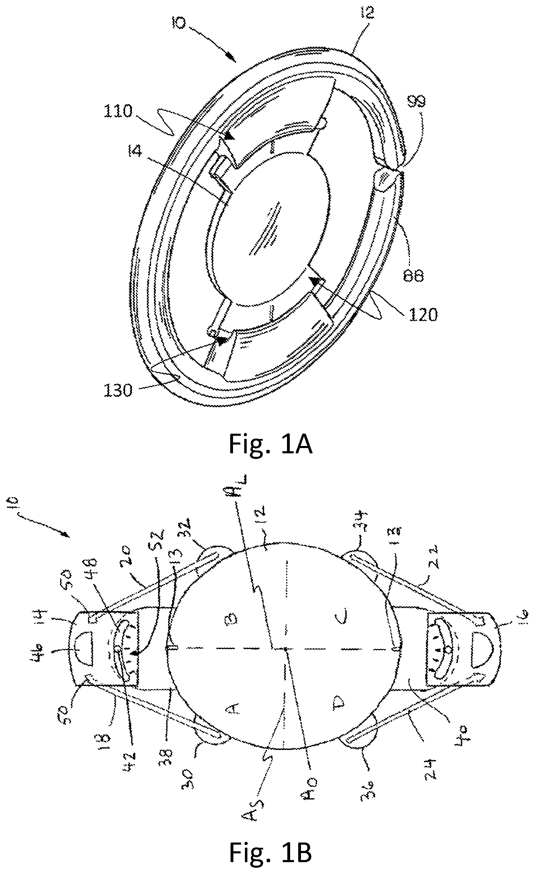

**Fig. 1A-D** shows examples of the prior art. In the prior art, a lens unique to the system must be used, with a connection between the lens and the support allowing rotation of the lens relative to the support.

In the embodiment of the prior art shown in **Fig. 1A****,** the lens **(14)** comprises two tabs **(120).** The support **(12)** also comprises tabs **(110),** these tabs having slots **(130)** into which the lens tabs **(120)** fit. In order to adjust the angular position of the lens **(14),** it is rotated manually, sliding the lens tabs **(120)** within the support tabs **(110).** The lens **(14)** is mounted into the support **(12)** by inserting the lens **(14)** with the lens tabs **(120)** in the open space between the support tabs **(110).** The lens **(14)** is then rotated until the lens tabs **(120)** are engaged with the support tabs **(110).** Therefore, the amount of angular adjustment is limited since there must be a gap between the support tabs **(110)** wider than the lens tabs **(120).**

In the embodiment of **Fig. 1B****,** the lens **(12)** is attached to the support by heat-shrinkable struts **(18, 20, 22, 24).** The lens **(12)** has tabs **(38, 40)** which can slide in the haptics (supports) **(14, 16).** Adjustment of angle is by either heat-shrinking one or more struts **(18, 20, 22, 24)** or by breaking one or more struts **(18, 20, 22, 24).** Therefore, the amount of adjustment is limited by the shrinkability and breakability of the struts **(18, 20, 22, 24)** and number of times the angular position of the lens (12) can be changed is also limited, since there appears to be no way of replacing a broken strut or stretching a shrunk strut. Furthermore, the lens of the embodiment of **Fig. 1B** does not appear to be replaceable, as there does not appear to be any way to replace a broken strut or to attach a strut to a new lens.

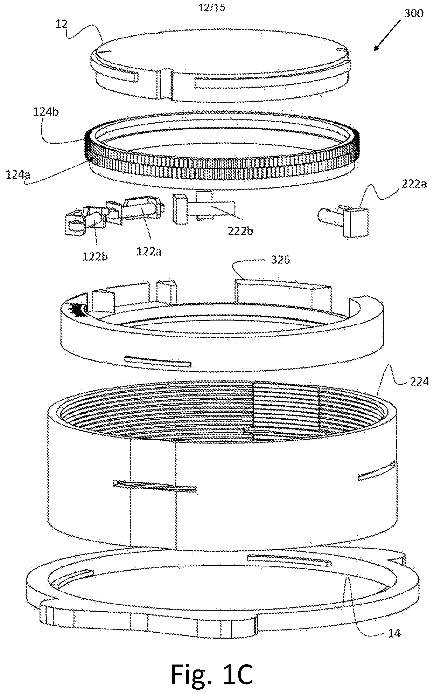

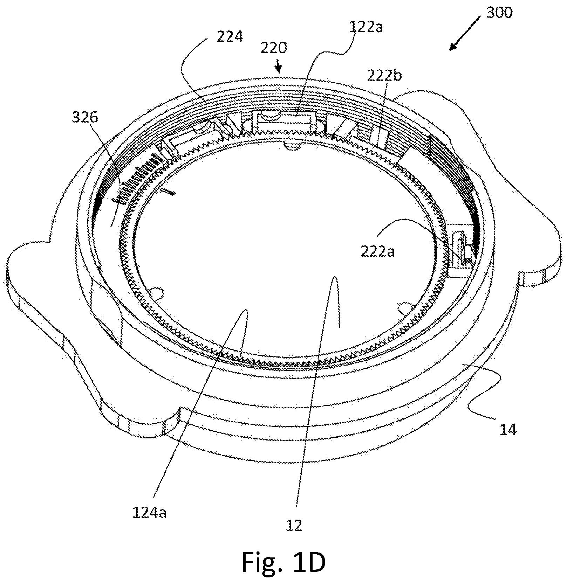

In the embodiment of **Fig. 1C-D****,** **Fig. 1C** shows an exploded view of the device **(300).** The haptics housing **(14)** holds an outer tooth arrangement mechanism (step driving mechanism, **224)** with vertically-disposed teeth for linear movement of the lens **(12).** The outer tooth arrangement mechanism **(224)** is removable from the haptics housing **(14)** and is held in the haptics housing **(14)** by means of a tooth and slot. Inside the outer tooth arrangement mechanism **(224)** is an actuator housing **(326)** with actuators **(122a, 122b, 222a, 222b),** as described below in reference to **Fig. 1D****.** Inside the actuator is a second, inner, tooth arrangement mechanism **(124a, 124b)** with horizontally disposed teeth for rotational movement of the lens **(12).** The lens **(12)** is a proprietary lens with a holding ridge on its periphery. The lens is permanently mounted by its holding ridge in a groove in the inner tooth arrangement mechanism **(124a, 124b).**

**Fig. 1D** shows the haptics housing **(14)** of the device (300) and a repositioning assembly **(220).** The repositioning assembly **(220)** comprises an actuator housing **(326)** comprising actuators **(122, 222a, 222b).** The actuators are configured to move a tooth arrangement mechanism (step driving mechanism, **124a, 224).**

For the prior art of **Figs. 1C-D****,** the only possible motions of the lens are (1) linear motion parallel to the optic axis of the lens and rotation motion about the optic axis of the lens. Decentralization, tilting and angulation are not possible.

It should be noted that, because of the necessarily small size of the device of **Fig. 1C-D** and its complexity, it would be difficult to build it and, once built, to control it. Furthermore, lasers are used to control the shape memory alloy of the actuator mechanism. It would be very difficult to get a good line of sight for the laser to the shape memory alloy, heating of the material within the eye by the laser would affect both the aim and the focus of the laser, and heating of the eye material by the laser can damage the eye.

It should further be noted that the device of **Fig. 1C-D** is rigid. There does not appear to be any flexible portion that would allow insertion of the device into the eye.

In the prior art, solutions to enable post-implantation correction of IOL lens positioning are based on a specifically designed lens or lenses which must be used with a matching support structure specific to that lens or lenses and post-operative adjustment of the rotation of the lens is either severely restricted or requires a second operation. Such devices cannot combine conventional lenses and enable the ability for post-operative remote rotation.

The present invention discloses an intraocular ring system (IRS) that enables post-implantation movement of a generic, non-manufacturer or design specific "off-the-shelf" intraocular lens (IOL). Movement can be for example forward-backward movement of an IOL or a rotation of a toric IOL in the eye without the need for additional surgery.

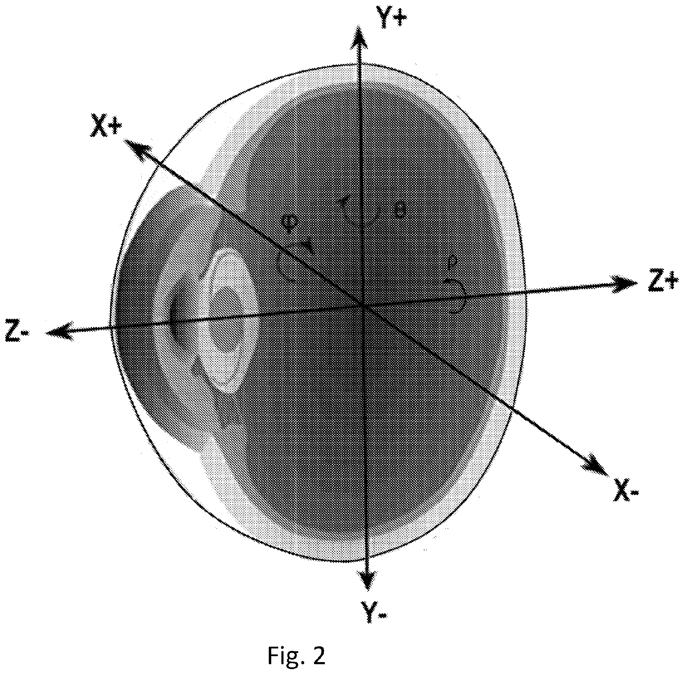

**Fig. 2** shows a typical coordinate system for an eye. The X axis is right-left through the eye. Looking from the cornea towards the retina, X- is at the right and X+ at the left. The Y axis is the inferior-superior axis, Y- being the inferior side of the eye (at the bottom for a standing person) and Y+ being the superior side of the eye (at the top for a standing person). The Z axis is the posterior-anterior axis, with Z- being the anterior (cornea) side of the eye and Z+ being the posterior (retina) side of the eye. Therefore, the X-Y plane will be a vertical plane from ear to ear in a standing person, the Y-Z plane will be vertical, from nose to nape of neck, and the X-Z plane will be horizontal in a standing person. The angle θ describes rotation about the Y axis, the angle φ describes rotation about the X axis and the angle ρ describes rotation about the Z axis.

An IRS will be aligned substantially in the X-Y plane. Forward-backward movement is along the Z axis, with forward movement being towards Z- and backward movement towards Z+. Decentering movement is in the X-Y plane, with rightward decentering being toward X- and leftward decentering toward X+.

"Rotation" is rotation in ρ, about the Z axis. "Tilt" is rotation in φ, about the X axis. Rotation in θ, about the Y axis, will be referred to as "angulation".

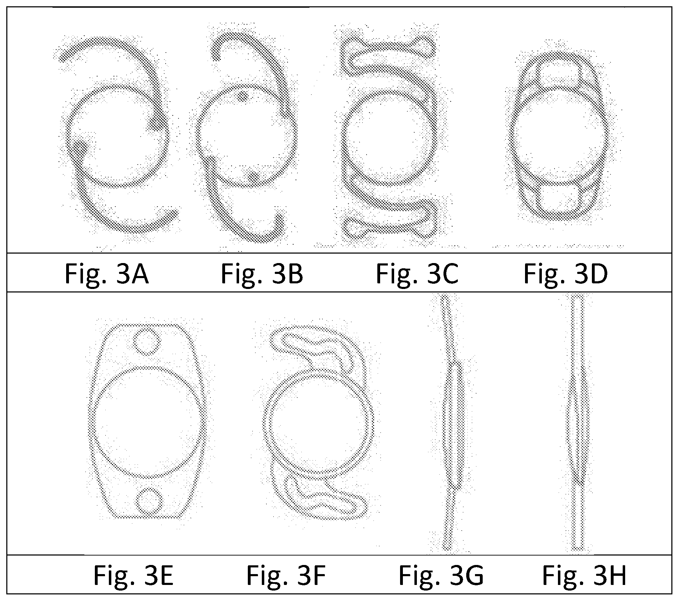

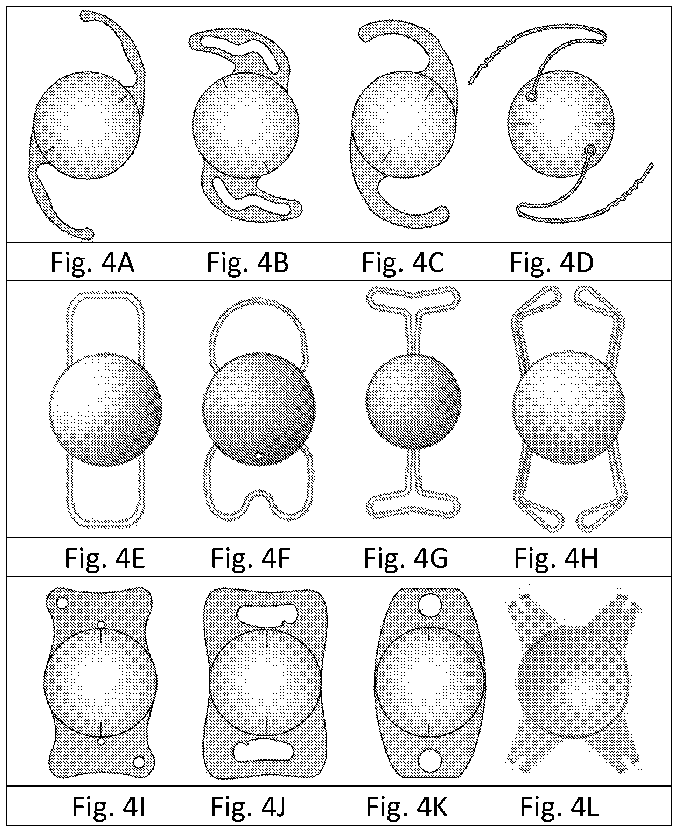

Examples of IOLs are shown in **Fig. 3A-H** and **Fig. 4A-L****.** **Fig. 3A-H** illustrates different types of IOL, while **Fig. 4A-L** illustrates IOLs in current use. **Fig. 3A** illustrates an IOL with C-loop haptics, **Fig. 3B** illustrates an IOL with J-loop haptics, **Fig. 3C** illustrates an angle supported anterior chamber (AC) IOL, **Fig. 3D** illustrates an iris-fixated IOL (with plate haptics), **Fig. 3E** illustrates an IOL with plate haptics, and **Fig. 3F** illustrates an IOL with plate loop haptics, while **Fig. 3G** illustrates an angular IOL and **Fig. 3H** illustrates a planar IOL. **Fig. 4A-D** illustrate IOLs with open loop arms, **Fig. 4E-H** illustrate IOLs with closed loop arms and **Fig. 4I-L** illustrate IOLs with plate arms. All of the IOLs shown are monoblock (single piece) IOLs.

The IRS of the present invention comprises a primary intraocular ring (PIR) and at least one secondary device (SD).

In the eye, the PIR is intended for permanent stationary placement in the capsular bag which is supported by the zonulas. Within the IRS, the PIR functions as a base for the one or more SDs to rotate upon while maintaining its stationary placement in the capsular bag.

In some embodiments, within the IRS, the SD sits upon the PIR. During IOL placement surgery, the IOL is placed upon the SD (and attaches to the SD) in the same precise manner as which the IOL is implanted in an eye without an IRS. By design the SD sits upon the PIR and can be non-surgically manipulated, moved and rotated in relation to the PIR. Minimal invasive procedures can also be used to move/rotate the SD. In case of multiple SDs, they may sit one inside the other or one next to another and serve distinct purposes.

In other embodiments, the SD (or the innermost SD) is attached to the IOL, with the IOL in contact with the PIR (or a next outer SD). The IOL can be non-surgically manipulated, moved and rotated in relation to the PIR. Minimally invasive procedures can also be used to move/rotate the IOL. In case of multiple SDs, they may sit one inside the other or one next to another and serve distinct purposes.

The first, outermost SD is attachable to the PIR and is supported by the PIR; each next SD, where present, is attachable to the previous SD, the SD next outward. SDs can have various shapes, structures and compositions. In use, in some embodiments, an IOL is attachable to and supported by the innermost SD. In embodiments with a single SD, the SD is attachable to and supported by the PIR and is attachable to and supports the IOL. In use, in some embodiments, the SD (or the innermost SD) is attached to the IOL, with the IOL in contact with the PIR (or a next outer SD).

In the eye, the PIR is intended for permanent stationary placement in the capsular bag, which is supported by the suspensory ligaments of the ciliary zonulas. Within the IRS, the PIR functions as a base for the SD to rotate upon; the PIR maintains its stationary placement in the capsular bag.

During IRS implantation surgery, the IOL is placed upon the SD (and is attached to or otherwise bonded with the SD); the SD and PIR are implanted in the eye in a similar manner as an IOL is implanted in an eye without an IRS. Implantation can be a multistep process - for non-limiting example, first implant a PIR, then attach an SD to the PIR, then attach the IOL to the SD; or it can be a one-step process, where the IRS is assembled, including all SDs, the PIR and the IOL, and the assembled IRS is implanted in the eye; and any combination thereof.

The SD can be rotated or moved (forward-backward or to the side) within the stationary PIR without need for further surgical intervention. Means of rotating or moving the SD (and the IOL) can comprise electricity, magnetism, ultrasound, chemicals, elastic forces, heat, potential energy, a laser, piezoelectricity or a combination of the above.

Since the IOL is bonded to the SD and the SD keeps the IOL separate from the capsular bag, there is a barrier between the IOL and the capsular bag of the eye, which eases post-implantation removal of the IOL, if such becomes necessary.

The PIR is manufactured from medical grade and implantation approved materials such as plastic, silicone, acrylic, metal, etc.

The SD is similarly manufactured from medical grade and implantation approved materials such as plastic, silicone, acrylic, metals, etc.

Advantages of the IRS include:
1. The IRS can support at least one conventional IOL and the IOL(s) and the IRS can be implanted in the eye during the same surgery. The IOL can be, but need not be, attached to the IRS before surgery.
2. The IOL suffers from less agglutination or fibrosis with the eye capsule than prior-art devices because of the two-element structure of the IRS, with a PIR supporting at least one secondary device (SD) and the at least one SD supporting the IOL. Therefore, removal of an implanted IOL, should that need arise post-implantation, is easier and more straightforward than with many prior-art devices.
3. The IRS can be designed to enable, post-implantation, at least one of forward-backward, (decentration and tilt movement of the implanted IOL. The ability to move the lens forward and backwards (also to tilt or decenter it) allows for improved positioning post-operation and adjusting of the patient's post-operative refraction and IOL alignment along the Z axis, if needed. In the prior art, post-surgical refinement of IOL placement, if possible at all, required additional surgical intervention such as IOL replacement, additional piggyback IOL implantation and laser corneal ablation.
4. The IRS can be designed to enable post-implantation rotation of implanted IOL(s) around the Z axis, without limitation on the size of the rotation or the number of rotation procedures. This allows for more precise and straightforward rotation of the IOL, and therefore better alignment with an astigmatic axis of an eye.
5. The IRS can be designed to allow precise and straightforward linear movement of the IOL, in at least one of the X direction, the Y direction, and the Z direction. In some embodiments, the IOL can be tilted relative to the PIR (and the eye) as well as the linear movement described above. These movements also allow improved post-operative uncorrected visual acuity for the patient since, in addition to the easier alignment of the IOL with a correction axis, there can be easier adjustment of the forward-backward location and the θ angle of the lens to improve the correction power of the lens.
6. Post-implantation rotation and movement of the IOL can be carried out without surgical intervention.
7. In the prior art, positioning of a toric IOL has been done with viscoelastic materials. When aspirating the viscoelastic material, the toric IOL can rotate. Such rotation may or may not be noticed by the surgeon and returning the IOL to the desired position likely requires use of more viscoelastic material. Viscoelastic material remaining behind the lens can also cause post-operative lens rotation. In addition, the accuracy of positioning tends to be low when viscoelastic materials are used.
8. The IRS can be designed to enable post-implantation non-concentric rotation of the implanted IOL. The ability to change the location of the optical axis of the IOL allows improved alignment of the IOL with a correction axis of the eye. This allows for more precise, straightforward and easier placement of the IOL along a correction axis and an improvement in the patient's post implantation vision. Prior to the introduction of the IRS, post-surgical refinement of IOL placement was imprecise and difficult and required additional surgical intervention.
9. The IRS can be designed to accommodate one or more slow drug release devices to augment or replace at least one type of post-operative eye drop and can also, under certain circumstances, replace or augment other post-operative medication. Types of eye drop can include, but are not limited to, a wetting eye drop, an analgesic, an antibiotic, and any combination thereof. A drug release device can also, for non-limiting example, allow for more refined dosage control and can improve user compliance.
10. The IRS can be designed to accommodate one or more drug release devices for treatment of chronic conditions such as glaucoma and macular degeneration.
11. The SD can have guides to ensure that the IOL haptics are positioned in the correct location and orientation and to prevent situations whereby the haptics are misplaced, for example between the capsular bag and the IRS or between the SD and PIR.
12. The PIR can have guides to ensure the IOL haptics are positioned to the correct location and to prevent situations whereby the haptics are misplaced, for example between the capsular bag and the IRS or between the SD and PIR.
13. The IRS can comprise sensors which are relevant to patient requirements (e.g., pressure sensor for glaucoma patients, glucose level within the anterior chamber for diabetic patients, etc.)
14. The IRS can comprise one or more cameras which can monitor retinal changes/abnormalities that might alert of any new pathologies, (e.g., hemorrhage, inflammation, drusen, abnormal flow patterns in retinal blood vessels etc.)

The IRS is designed to make use of current IOL surgical implantation knowledge and practices. During the course of IOL implantation surgery, the IRS will be inserted into the capsular bag in a manner similar to the manner in which an IOL is presently implanted. There are several possible implantation approaches:
1. The IOL and the IRS can be, but need not be, implanted through a common incision. The common incision can, but need not, have the same characteristics as current IOL implantation incisions.
2. The removal of the natural lens can be completed in the same manner as it is in situations where there is no IRS implantation.
3. The IRS and the IOL can be implanted as a unit with the IOL held by the IRS. The unit can be assembled prior to the surgery by a manufacturer, integrator, etc., by a member of the surgery team or surgery preparation team, or by another party.

The IRS and the IOL can also be implanted individually, as individual elements, in a defined order and by a defined process.
a. If the IRS and the IOL are implanted as individual elements, the IRS will be implanted prior to the IOL. After implantation of the IRS, the IOL will be implanted and placed onto the SD element of the IRS. The SD or PIR guides will ensure that the IOL haptics slide into the correct location and will help prevent IOL haptic misplacement.
b. During implantation, in the case of a toric IOL, the surgeon can set the axis of the IOL as near optimal as possible.

Prescribed drugs can be either slowly released by a device supported by the IRS or, as in the prior art, can be instilled using eye drops.

The SD is designed to allow itself and the IOL to be repositioned by at least one of: being moved forward and backward, being rotated and being decentered relative to the PIR (and the eye) post implantation to allow for better alignment of the IOL relative to the fovea. The ability to perform at least one of: moving the IOL backward and forward, rotating it, decentering it, angulating it and tilting it are features that can be utilized to adjust unexpected post-operative refractive outcome.

The need for better alignment of the IOL location in the eye will become known post implantation surgery during a standard post-operative refraction checkup. Should it be necessary to move the IOL, the SD and IOL can be moved as disclosed above via a non-surgical procedure which can be completed during an office visit.

There are a number of methods by which an IOL, an SD or both can be moved, rotationally, linearly forward-backward, decentralized, tilted, angulated or all five:
1. A small piece of magnetic material, a magnet or an electromagnet can be attached to or be part of an SD. Under the influence of a magnetic field, the magnetic material, magnet or electromagnet will exert force or torque on the SD which will cause it to move or rotate.
2. A small motor (electric, piezoelectric, twisted polymer or other) can be attached to or be part of an SD or be connected to the IRS, which moves the SD on receipt of an external command to move the SD.
3. A thermal actuator, SMA actuator, electro-reactive actuator, shape shift material actuator or other actuator, which is connected to the IRS, which moves the SD on receipt of an external command to move the SD.
4. Usage of ultrasonic energy to create an acoustic field which will cause rotation of the SD.
5. A heat reactive material, heat shrink plastic for example, which responds to laser energy and shrinks.
6. An elastic material or a spring which is held in a tensioned or compressed state by a device which can be removed or altered to allow the elastic energy to be released.
7. Geometric translation of rotation to linear, front-back, for example, movement by a screw-like geometry.
8. The energy to rotate the SD can be stored inside the eye in the lens system, for example elastic energy in a pretensioned band or chemical energy in a micro battery, or transmitted from outside the eye to the lens system, for example by laser energy, ultrasound energy or electric or magnetic induction.

The SD is designed to allow movement of itself and the IOL post implantation to allow for better alignment of the IOL to the required optical correction for the eye. The movement can be rotation, forward-backward movement, tilt, decentration, angulation and any combination thereof.

It is anticipated that the ability to rotate the IOL, either concentrically, non-concentrically, or both, is a feature that will be utilized primarily when an IOL that includes a toric correction is implanted. During a standard post operation vision check the need for better alignment of the IOL with the angle and side-to-side position of the correction axis of the eye and to a forward-backward location to ensure optimal focusing can be determined. Should movement be necessary, the IOL/SD can be manipulated to improve alignment of the angle and side-to-side position of the correction axis and to improve focusing by adjusting the forward-backward location. Manipulation of the IOL/SD is a non-surgical procedure which can be completed during an office visit.

Additionally, non-concentric rotation of the SD relative to the PIR can also be achieved to better align lenses to the patient's needs.

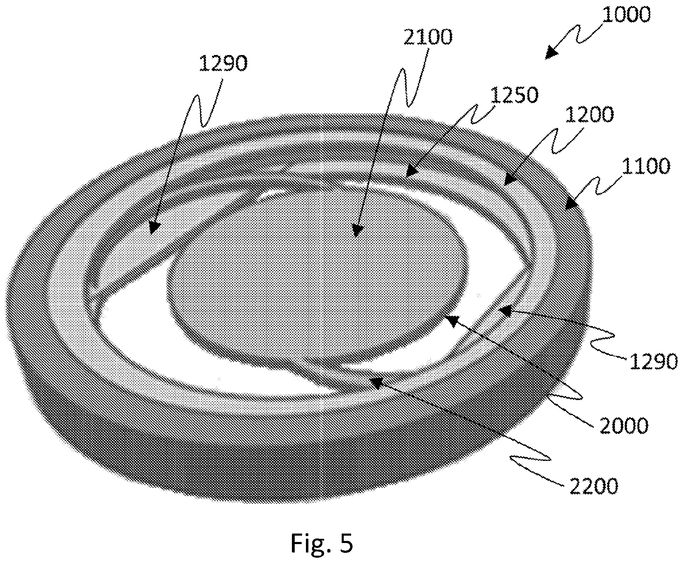

**Fig. 5** schematically illustrates an IRS **(1000)** of the present invention, with an IOL **(2000)** in position. The PIR **(1100),** which, in use, is supported by the capsular bag of the eye (not shown), holds an SD **(1200).** The haptics **(2200)** of the IOL **(2000)** are held in a mounting groove **(1250)** in the SD **(1200).** In the embodiment shown, the IOL **(2000)** is of a conventional form, with a central refractive portion (optic) **(2100)** and the two haptics are flexible arms **(2200).** In other conventional IOLs (not shown) the haptics can be plates, arms and any combination thereof.

It is clear that any IOL with at least two haptics, at least one of them being flexible, could be used as an IOL in the IRS **(1000)** of the present invention. The IOL guides **(1290),** which guide the IOL haptics to the correct location and prevent situations where the haptics are misplaced, for example, placed between the capsular bag and the IRS, are visible below the extremities of the haptics.

In preferred embodiments, the SD is removably held by the PIR. In some embodiments, the SD is permanently held by the PIR. In all embodiments, the SD is movable with respect to the PIR. The movement can be in a direction, it can be a rotation, or both, as described herein.

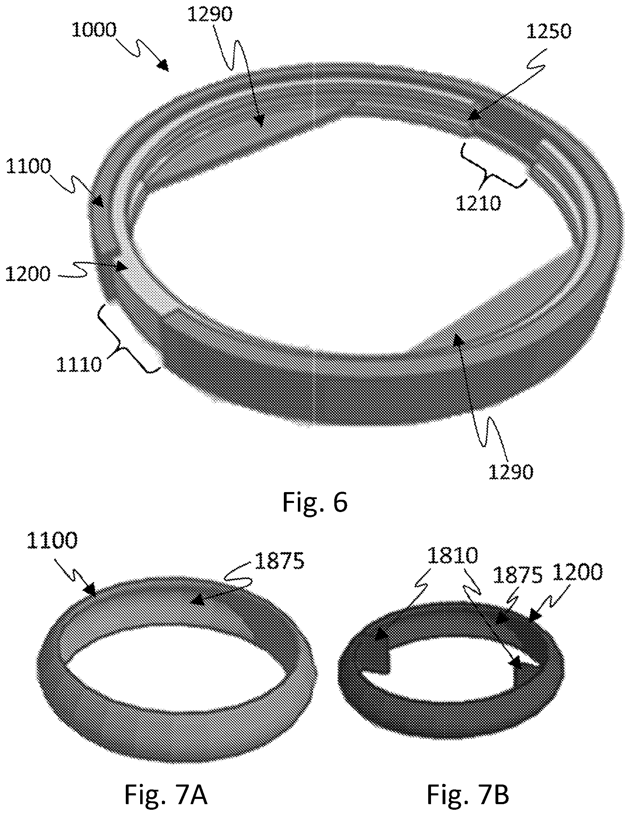

**Fig. 6** schematically illustrates another embodiment of the PIR **(1100)** and SD **(1200),** shown for clarity without an IOL. In this embodiment, the PIR **(1100)** and the SD **(1200)** are of rigid material and are partial rings, each comprising a gap **(1110, 1210)** to allow for adjustments in diameter, the adjustments in diameter allowing, on the one hand, compression so that the PIR and/or SD can be inserted into the eye and, on the other hand, adaptation of the PIR and/or SD to eyes of different sizes. The SD **(1200)** has a mounting groove **(1250)** to accommodate the intraocular lens (not shown) and to hold the intraocular lens firmly. IOL guides **(1290)** are shown.

For PIR and SD with gaps, one adult size is needed; for flexible PIR and SD, 3 adult sizes are needed and one child size. For some embodiments of PIR and SD with gaps, a child size is needed. **Fig. 7** schematically illustrates an embodiment of an IRS **(1000)** of the present invention. The PIR **(1100)** and SD **(1200)** have a U-shaped cross-section **(1875).** In the embodiment of **Fig. 7****,** the SD **(1200)** is rotatable via shaped ultrasonic fields acting on tabs **(1810).** In this embodiment, there are a pair of tabs **(1810).**

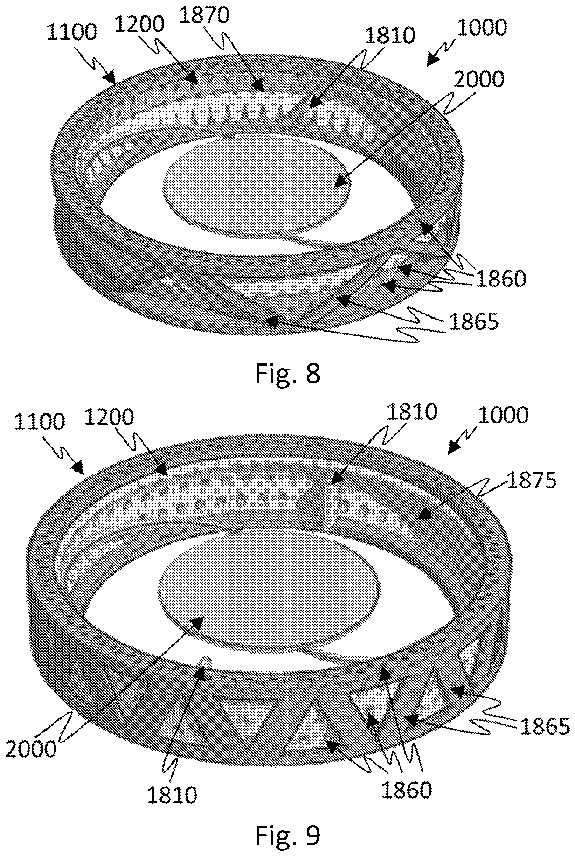

**Fig. 8** schematically illustrates an embodiment of an IRS **(1000)** of the present invention, with an IOL **(2000)** in position. The PIR **(1100),** which, in use, is supported by the capsular bag of the eye (not shown), holds an SD **(1200).** The side of the SD **(1200)** is V-shaped, with the haptics of the IOL **(2000)** held in place by the V groove **(1870)** formed by the point of the V.

The holes **(1860)** and bars **(1865)** reduce the weight of the PIR **(1100)** and SD **(1200)** without significantly affecting their strength or rigidity. Any combination of holes and bars can be used; each of the PIR **(1100)** and SD **(1200)** can have holes, bars and any combination thereof.

In the embodiment of **Fig. 8****,** the SD **(1200)** is rotatable via shaped ultrasonic fields acting on tabs **(1810).** In this embodiment, there are a pair of tabs **(1810);** only one is visible.

**Fig. 9** schematically illustrates an embodiment of an IRS **(1000)** of the present invention, with an IOL **(2000)** in position. The PIR **(1100),** which, in use, is supported by the capsular bag of the eye (not shown), holds an SD **(1200).** The side of the SD **(1200)** is U-shaped, with the haptics of the IOL **(2000)** held in place near the deepest portion of the U **(1875).**

In the embodiments of **Figs. 8** and **9****,** the IOL **(2000)** is of a conventional form, with a central refractive portion (optic) and two flexible haptics. In other IOLs (not shown) mountable in the IRS **(1000)** of the present invention the haptics can be plates, arms and any combination thereof. Any IOL with at least two haptics, at least one of them being flexible, could be used as an IOL in the IRS **(1000)** of the present invention.

The holes **(1860)** and bars **(1865)** reduce the weight of the PIR **(1100)** and SD **(1200)** without significantly affecting their strength or rigidity. Any combination of holes and bars can be used; each of the PIR **(1100)** and SD **(1200)** can have holes, bars and any combination thereof.

In the embodiments of **Figs. 7- 9** the SD **(1200)** is rotatable via shaped ultrasonic fields acting on pairs of tabs **(1810).** In the embodiment of **Fig. 8****,** only one tab **(1810)** is visible.

If the ultrasonic field is configured to apply pressure on tabs in opposite directions, the pressure rotates the SD. If the ultrasonic field is configured to apply pressure on tabs in the same direction, the pressure decenters the SD, with the amount of decentration limited by the size of the gap between the SD and the PIR.

If the ultrasonic field is configured to apply a greater pressure on an upper portion of a tab (outward from the eye) than a lower portion of the tab (inward from the eye), or vice versa, the SD (and the IOL) can be tilted, angulated and any combination thereof, depending on the location of the tab(s) and the shape of the ultrasonic field, with the amount of tilt or angulation limited by the size of the gap between the SD and the PIR.

Decentration, tilt and angulation can similarly be accomplished by varying the magnitudes of the forces applied to accepting objects by motors or by shaped magnetic fields, where the motors, magnetic fields and accepting objects are disclosed herein.

In preferred embodiments, there are pairs of tabs, with the number of pairs of tabs being in a range from 1 to 15. In less-preferred embodiments, tabs are not paired and the number of tabs is in a range from 1 to 30 or 2 to 30.

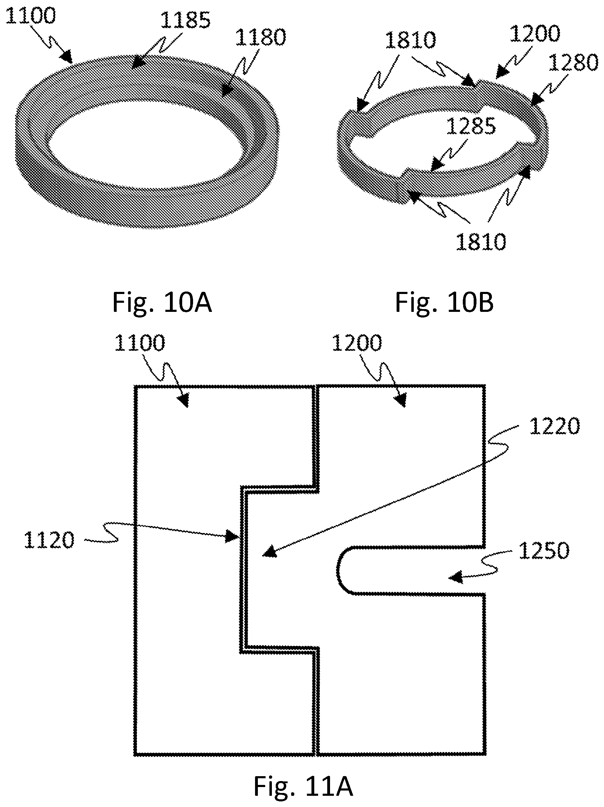

**Fig. 10A-B** schematically illustrates an embodiment of an IRS **(1000)** of the present invention. In this embodiment, the PIR **(1100)** is stepped, with the SD **(1200)** configured to rest in the step **(1180)** and to slide on the riser **(1185)** of the step. In this embodiment, the two larger-diameter sections **(1280)** of the SD are circular, with an outer diameter corresponding to the inner diameter of the step riser **(1185)** so that the SD **(1200)** rests on the step **(1180)** and slides against the step **(1180)** and the step riser **(1185).** The SD **(1200)** is rotatable via shaped ultrasonic fields acting on the tab regions **(1810).** The smaller sections **(1285)** joining the tab regions **(1810)** can be any convenient shape such as partial circles, partial ovals, partial ellipses, liner, partial polygons and any combination thereof; as shown, the smaller sections **(1285)** form sections of a circle of smaller diameter than the larger-diameter sections **(1280).**

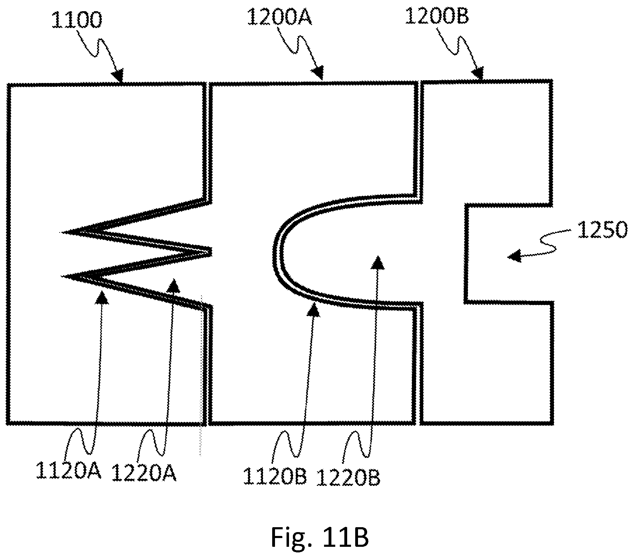

**Fig. 11A-B** schematically illustrates cross-sections of embodiments of the PIR **(1100)** and SD(s) **(1200).** In these exemplary embodiments, the PIR **(1100)** comprises a holding groove **(1120)** and the SD **(1200)** comprises a matching tongue **(1220).** As disclosed above, in other embodiments, the PIR-SD connection can comprise a U-shape, a C shape, a V shape, a zigzag, a truncated V a truncated C and any combination thereof. In any embodiment, the PIR-SD connection allows both a robust connection between the PIR **(1100)** and the SD **(1200)** and ensures that the SD **(1200)** can be reliably rotated within the PIR **(1100).** In some embodiments, the SD **(1200)** comprises a holding groove **(1120)** and the PIR **(1100)** comprises a matching tongue **(1220).** The SD comprises a mounting groove **(1250)** or other SD-haptic connection, as disclosed herein, to hold a haptic or plate of the IOL. **Fig. 11A** schematically illustrates a tongue and groove of rectangular cross-section, while **Fig. 11B** schematically illustrates two different types of cross-section, V-shaped or zigzag **(1120A, 1220A)** and U-shaped **(1120B, 1220B).** The PIR-SD connection can be intercalated, overlapping, interlocking and any combination thereof.

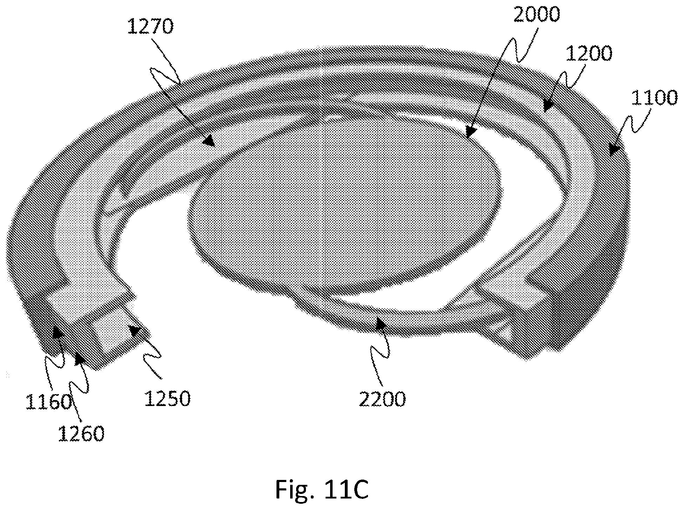

**Fig. 11C** schematically illustrates a cut-away view of an embodiment of the PIR **(1100)** and SD **(1200).** In this embodiment, the inner face **(1160)** of the PIR **(1100)** and the outer face **(1260)** of the SD **(1200)** are U-shaped, with the outer face **(1260)** of the SD **(1200)** smoothly and controllably slidable against the inner face **(1160)** of the PIR **(1100).** The haptics **(2200)** of the IOL **(2000)** are shown, resting on the IOL guides **(1270)** and within the mounting groove **(1250).**

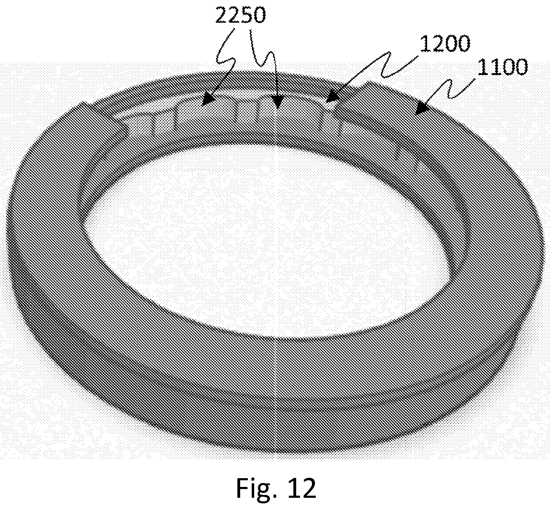

**Fig. 12** schematically illustrates a cut-away view of an embodiment of the PIR **(1100)** and SD **(1200)** configured to allow stable positioning of the IOL in the IRS. The IOL **(2000)** is not shown. In this embodiment, the inner face of the SD **(1200)** comprises indentations **(2250)** configured to hold the haptic(s) of an IOL stably in position. In the embodiment as shown, the indentations **(2250)** have the shape of sections of a right circular cylinder. Indentations can have the shape of a cap of a sphere, a cap of an ellipsoid, triangular, a cuboid, a section of a right cylinder, a section of an oblique cylinder and any combination thereof or even a rough surface or one with adequate friction. The cylindrical indentation can have a circular cross-section, an ellipsoidal cross-section or a polygonal cross-section. In embodiments of this type, in use, the distal end of each haptic rests in an indentation, thereby preventing undesired rotation of the IOL; rotation of the IOL is by rotating the SD **(1200),** or by transferring each distal end of each haptic from its present indentation to another indentation.

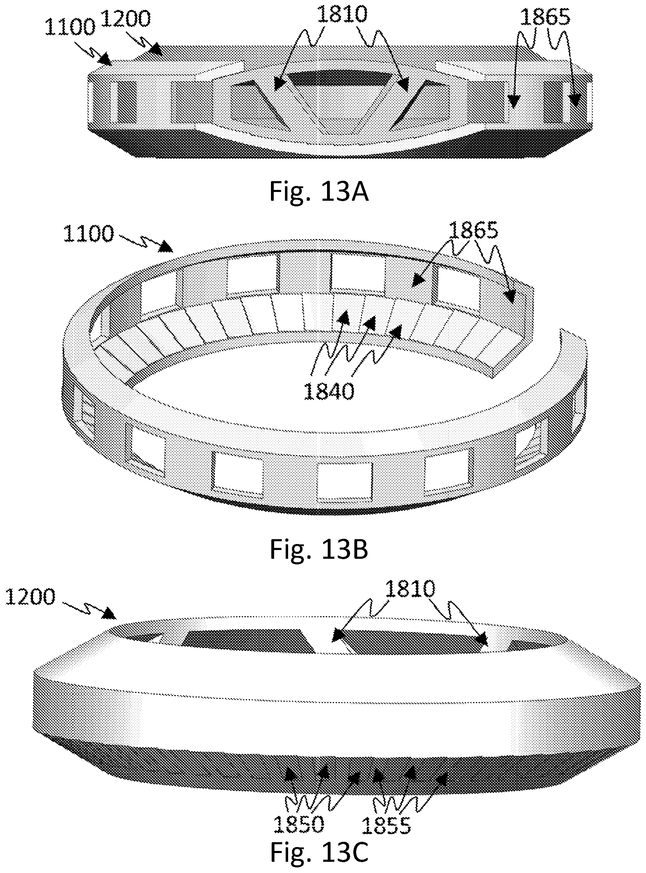

**Fig. 13A-C** schematically illustrates an embodiment of the PIR **(1100)** and SD **(1200)** of an IRS. **Fig. 13A** shows the SD **(1200)** in position inside the PIR **(1100),** **Fig. 13B** shows the PIR **(1100)** and **Fig. 13C** shows the SD **(1200).** In the embodiment of **Fig. 13A-C****,** the PIR **(1100)** has bars **(1865)** to reduce the weight of the structure. The diagonal bars **(1810)** of the SD **(1200)** function both as supports and as tabs to accept ultrasonic fields and thereby to rotate the SD **(1200)** relative to the PIR **(1100).** In the variant of this embodiment which is illustrated, the amount of rotation is altered in a step-like manner by means of a ratchet-like mechanism; in other variants of this embodiment, any other mechanism, as disclosed herein, for rotating the SD relative to the PIR can be used.

The PIR **(1100)** comprises ratchet-like steps **(****Fig. 13B****, 1840)** with a slow rise and a sharp drop. There are similar ratchet-like steps **(****Fig. 13C****, 1850)** on the SD **(1200),** separated by bars **(1855)** to improve device stability. When the ultrasonic fields act on the bar tabs **(1810),** the SD **(1200)** is rotated until the SD ratchet **(1840)** snaps to a next PIR ratchet **(1850)** position, thus ensuring that there is a fixed amount of rotation per step.

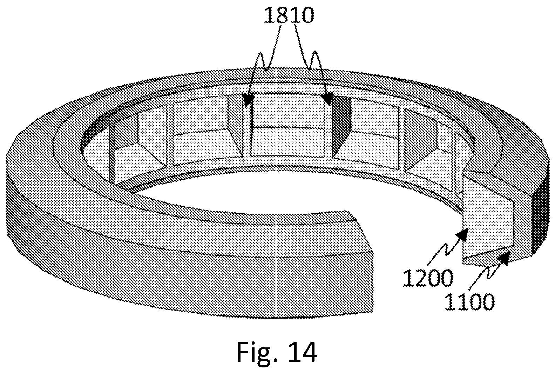

**Fig. 14** schematically illustrates an embodiment of the PIR **(1100)** and SD **(1200)** of an IRS. In this exemplary embodiment, the vertical bars **(1810)** of the SD **(1200)** function both as supports and as tabs to accept ultrasonic fields and thereby to rotate the SD **(1200)** relative to the PIR **(1100);** in this embodiment there are 8 pairs of vertical bars **(2410).** The PIR **(1100)** has a truncated V inner profile, matching the truncated V outer profile of the SD **(1200).**

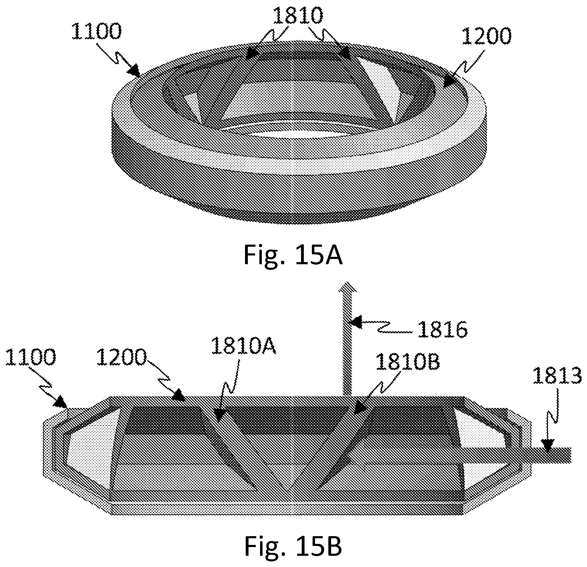

**Fig. 15A-B** schematically illustrates an embodiment of the PIR **(1100)** and SD **(1200)** of an IRS. **Fig. 15A** shows a perspective view, while **Fig. 15B** shows a cross-section. The PIR **(1100)** has a truncated V inner profile, matching the truncated V outer profile of the SD **(1200).** In the embodiment of **Fig. 15A-B****,** diagonal bars **(1810A, 1810B)** of the SD **(1200)** function both as supports and as tabs to accept ultrasonic fields and thereby to rotate the SD **(1200)** relative to the PIR **(1100).** In an embodiment of this type, with diagonal bars **(1810A, 1810B)** or diagonal tabs (not shown) a force **(1813)** applied to the diagonal bars **(1810A, 1810B)** or diagonal tabs (not shown) will cause the SD **(1200)** to rotate relative to the PIR **(1100)** and the eye. Since the force **(1813)** is at an angle to the diagonal bars **(1810A, 1810B)** or diagonal tabs (not shown), the force **(1813)** will also cause the SD **(1200)** to move linearly **(1816)** relative to the PIR **(1100)** and the eye. In embodiments such as the exemplary embodiment of **Fig. 15A-B** where diagonal bars **(1810A, 1810B)** or diagonal tabs (not shown) are angled both forwardly **(1810B)** and backwardly **(1810A),** linear motion in both directions is possible, thereby allowing correction of focus, tilt and angulation as well as correction of the toric angle,

It should be noted that any of the embodiments herein can be made with holes **(1860)** and bars **(1865)** to reduce the weight, as disclosed hereinabove.

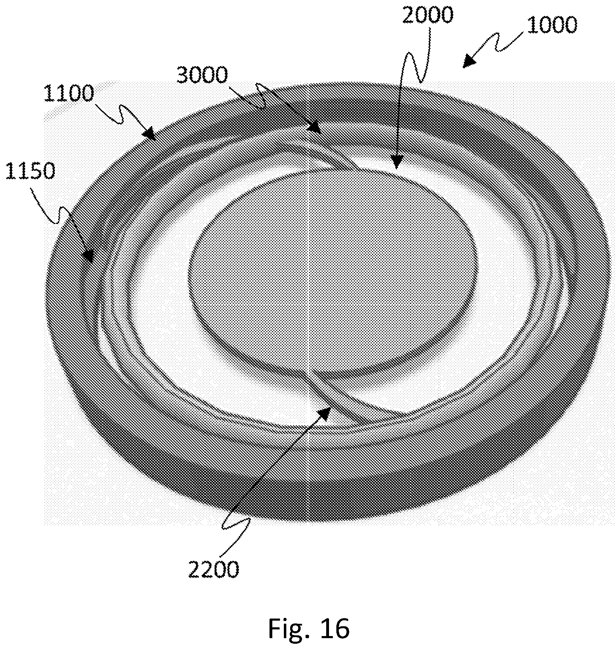

In some embodiments, the SD is connected directly to the IOL, but the SD is not connected to the PIR or to another SD. **Fig. 16** schematically illustrates an embodiment of the IRS **(1000)** with one SD **(3000),** where the SD **(3000)** is connected directly to the IOL **(2000)** and the IOL **(2000)** is connected to the PIR **(1100),** but the SD **(3000)** is not connected to the PIR **(1100).** The haptics **(2200)** are connected to the SD **(3000),** with the distal ends of the haptics **(2200)** being held by the PIR **(1100).** In the embodiment shown in **Fig. 16****,** the distal ends of the haptics **(2200)** are held in a groove **(1150)** in the PIR **(1100).**

In embodiments where the SD **(3000)** is connected to the IOL **(2000)** but not to the PIR **(1100),** the PIR **(1100)** serves as a device to eliminate contact and friction between the IOL **(2000)** and the capsular bag or zonulas.

In some variants of the embodiments disclosed above with an SD connected only to the IOL, at least one SD is intermediate between the IOL and the PIR. In such variants, an innermost SD is connected to the IOL but not to another SD and there is at least one additional SD, the additional SD(s) being intermediate between the IOL and the PIR with the IOL being held directly by an innermost of the additional SD(s).

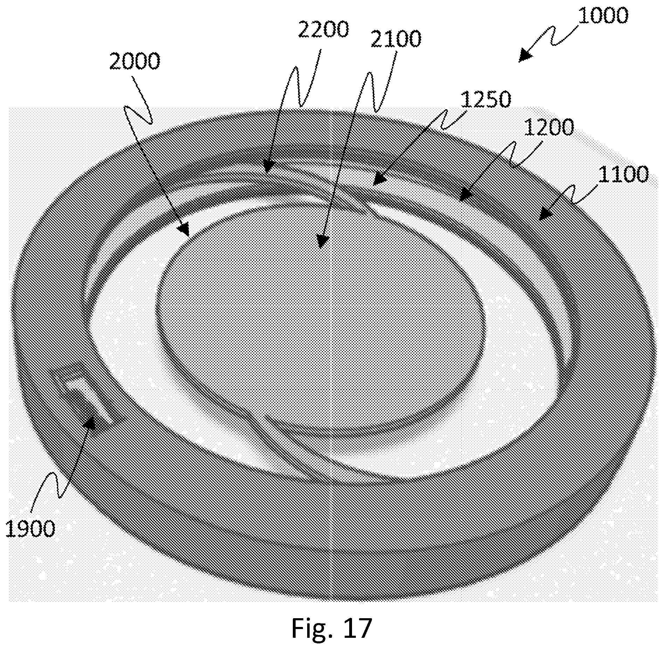

**Fig. 17** schematically illustrates another embodiment of the IRS **(1000),** showing the PIR **(1100)** and SD **(1200),** with the IOL **(2000)** in place, the lens **(2100),** in the center of the system, held in place by the two haptics **(2200),** which are robustly held in the mounting groove **(1250).** In this embodiment, an elastic energy movement mechanism **(1900)** enables rotation of the SD **(1200).**

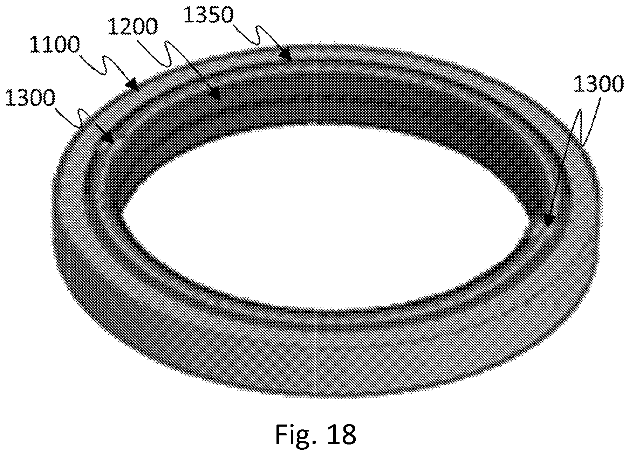

**Fig. 18** schematically illustrates another embodiment of the PIR **(1100)** and SD **(1200).** The SD **(1200)** is supported by the PIR **(1100);** in this embodiment the PIR **(1100)** and the SD **(1200)** have the shape of a ring. To enable non-invasive angular adjustment of the SD **(1200),** the SD **(1200)** comprises a magnet, an electromagnet **(1300),** a region of magnetic material and any combination thereof movable by an externally generated magnetic field, to enable torque to be applied to the SD **(1200)** to rotate it. In the embodiment as shown, there are two magnets or electromagnets **(1300).** In embodiments in which at least one electromagnet **(1300)** is used, the SD **(1200)** can further comprise an induction ring **(1350)** to allow the electromagnet(s) **(1300)** to be powered from a remote source, thereby ensuring that the at least one electromagnet **(1300)** is unpowered unless an angular adjustment is to be performed and the power is needed.

Magnetic material can comprise, but is not limited to, iron, nickel, cobalt, alnico, a ceramic ferrite, samarium, cobalt, neodymium and any combination thereof.

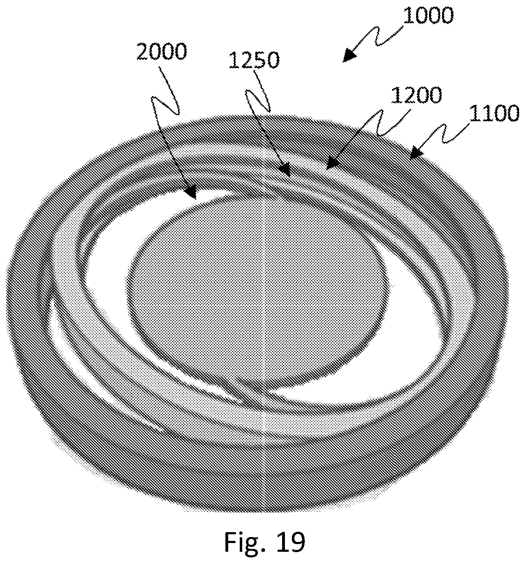

**Fig. 19** schematically illustrates another embodiment of the IRS **(1000)** which comprises a PIR **(1100)** and an SD **(1200).** An intraocular lens **(2000)** is shown, mounted in the mounting groove **(1250)** in the SD **(1200).** In this embodiment, the SD **(1200)** is oval rather than circular, which allows for greater rotational stability for the IOL. In this embodiment, the SD **(1200)** is not coplanar with the PIR **(1100),** allowing angular adjustment of the intraocular lens **(2000)** about two axes, on parallel to the plane of the PIR **(1100)** and one perpendicular to the plane of the PIR **(1100).**

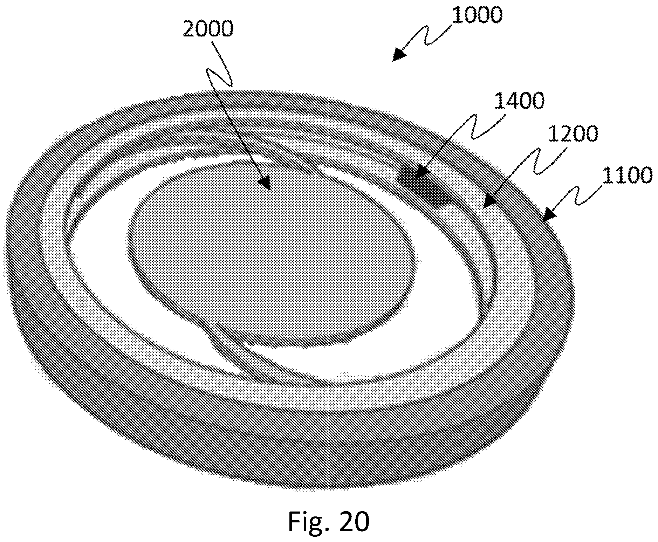

**Fig. 20** schematically illustrates another embodiment of the IRS **(1000)** comprising a PIR **(1100)** and SD **(1200).** An intraocular lens **(2000)** is shown. The SD **(1200)** comprises a motor **(1400)** to enable the SD **(1200)** to be controllably and non-invasively rotated within the PIR **(1100).** The motor can be an electric motor, a piezoelectric motor, a sonic motor, a twisted polymer or any other miniature motor. Power for the motor can supplied from an external source, such as, for non-limiting example, via the induction ring disclosed hereinabove via a battery and any combination thereof.

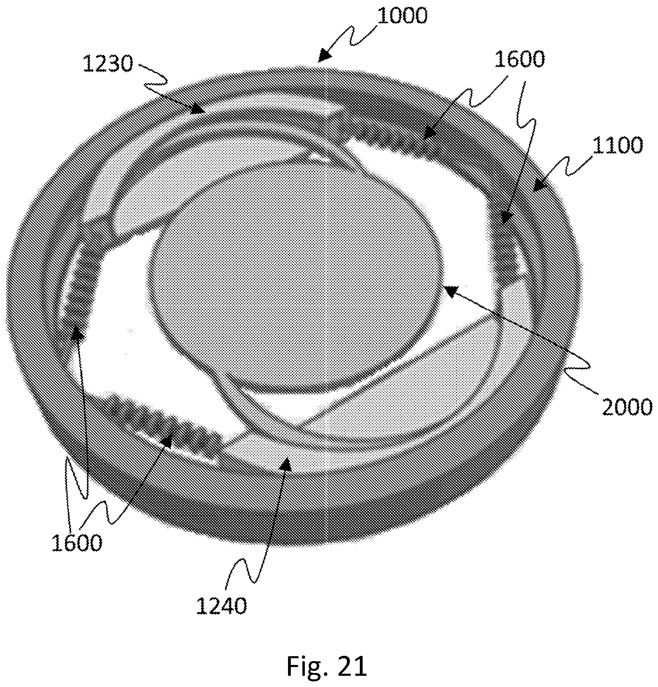

**Fig. 21** schematically illustrates another embodiment of the IRS **(1000)** comprising a PIR **(1100)** and SD **(1230, 1240).** The IOL **(2000)** is shown. The SD **(1230, 1240)** is divided into a plurality of parts **(1230, 1240)** each of which is connected to the PIR **(1100)** by at least a portion of an elastic band or spring **(1600).** In the embodiment shown, each part is connected to the PIR (1100) by two elastic bands or springs **(1600).**

The elastic bands or springs **(1600)** are pretensioned to provide energy to rotate the portion of the SD **(1230, 1240)** to which they are connected. In the embodiment shown, there are four elastic bands or springs **(1600).** In other variants of this embodiment, more or fewer elastic bands or springs **(1600)** can be used. A combination of elastic bands and springs can also be used.

In some embodiments, one or more pairs of springs or elastic bands can comprise a single spring or elastic band, with either the ends of the spring or elastic band attached to the PIR and the center of the spring or elastic band attached to the SD or the ends of the spring or elastic band attached to the SD and the center of the spring or elastic band attached to the PIR.

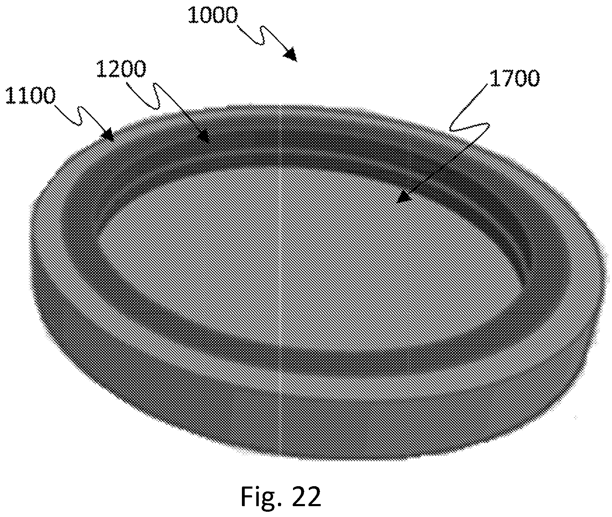

**Fig. 22** schematically illustrates another embodiment of the IRS **(1000)** comprising a PIR **(1100)** and SD **(1200).** In this embodiment, the PIR **(1100)** has a solid back **(1700);** instead of comprising an open ring, the PIR has substantially the shape of an open-topped cylinder, with the sides of the cylinder having the shape of the open ring of the embodiments shown hereinabove and the closed base of the cylinder, the solid back **(1700)** of the PIR (1100), facing the interior of the eye.

A back **(1700)** can prevent the posterior capsule of the eye from adhering to the IOL **(2000,** not shown), thereby enabling easy IOL **(2000,** not shown) rotation/movement even months or years after implantation of the IRS **(1000),** and minimizes difficulty in replacing the IOL **(2000,** not shown), even months or years after implantation of the IRS **(1000).**

The back is typically at least partly transparent.

In some embodiments that comprise a back, the back **(1700)** is not solid. A non-solid back can have perforations, slots, holes or other regularly or irregularly shaped gaps, can comprise a mesh, and any combination thereof. The back can also cover only a part of the area within the ring.

In some embodiments, the IRS comprises an element such as a sensor, a camera, a source of medication, and any combination thereof. The sensor can be for pressure, glucose, oxygen, CO₂, a chemical sensor to determine an amount of medication present, and any combination thereof. The medication source can comprise a steroid, a corticosteroid, an antibiotic, a nonsteroidal antiinflammatory drugs (NSAID) and any combination thereof. The medication source can reduce or eliminate the need to administer medications such as, but not limited to, eye drops after the operation. It can slowly release one or more medications into the chamber of the eye to help the patient heal and to prevent infections.

In some embodiments, more than one IOL can be placed in the mounting groove of an SD; in some embodiments, independent rotation of the IOLs in the mounting groove is allowed.

In some embodiments, the IRS can be configured to allow a second IOL to be implanted independently of the first IOL. This can allow easier adjustment of the optical power of the IOL. In such embodiments, the IRS can be deeper (along the Z axis) than embodiments configured for only a single lens; the PIR can be configured with two SDs; the SD can be configured with two mounting grooves, each configured to hold a single IOL; and any combination thereof.

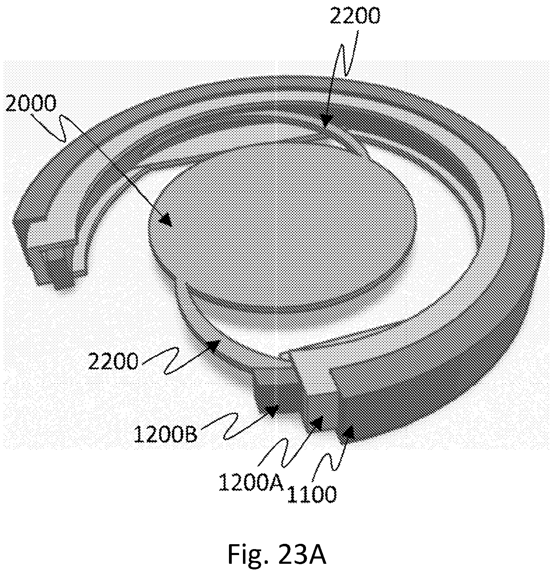

As schematically illustrated in **Fig. 23A-D****,** in some embodiments, the IRS can comprise two or more SDs **(1200A, 1200B),** which can be movable relative to each other or can be fixed relative to each other. Typically, in use, the IOL **(2000)** is removably attached to the innermost SD **(1200B)** by its haptics **(2200)** and can be rotated relative to the innermost SD **(1200B).** In the embodiments shown in **Fig. 23A-C****,** the innermost SD **(1200B)** is then movably attached to an outer SD, which is movably attached to the PIR **(1100)**

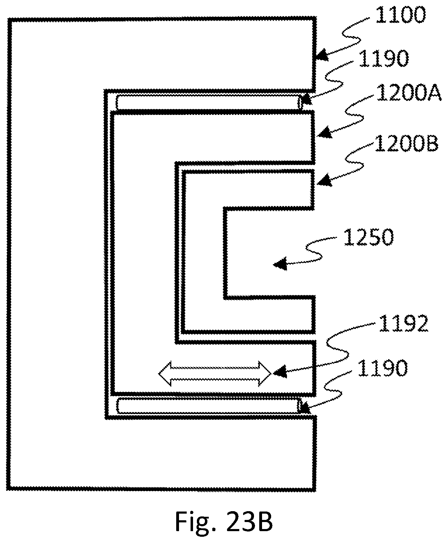

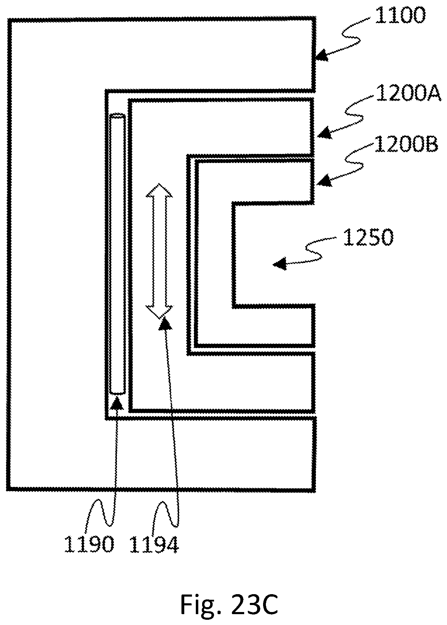

**Fig. 23A** shows a cutaway view of an embodiment of an IRS with two SDs **(1200A, 1200B)** while **Figs. 23B** and **23C** schematically illustrate cross-sections of embodiments of the IRS with screw-type adjustment mechanisms.

As schematically illustrated in **Fig. 23B****,** one SD **(1200B)** allows toric (rotational) adjustment to align the IOL with the correction axis, and one SD **(1200A)** allows decentering **(1192)** adjustment to improve alignment of the cylinder axis with the astigmatic axis of the eye.

As schematically illustrated in **Fig. 23C****,** one SD **(1200B)** allows toric (rotational) adjustment to align the IOL with the correction axis, and one SD **(1200A)** allows forward-backward location adjustment **(1194)** via an adjustment mechanism **(1190),** to adjust the optical power of the IOL. The adjustment mechanism **(1190)** can comprise a screw, a ratchet, a magnetic region, a magnetic tab, a tab to accept ultrasonic waves, and any combination thereof.

The SDs for toric adjustment can be, but need not be, concentric with the SDs for forward-backward location adjustment. In the embodiments of **Fig. 23A-B****,** the outer SD **(1200A)** is configured for forward-backward or decentering adjustment and the inner SD **(1200B)** is configured for toric (rotational) adjustment. However, the various adjustments can be in any order. An adjustment mechanism **(1190)** can be one or more miniature screws (as illustrated in **Fig. 23B-****C),** a screw-like geometry of the PIR **(1100),** a screw-like geometry of the SD, shape memory alloy, a miniature motor, as disclosed above, and any combination thereof.

In embodiments where the IRS **(1000)** comprises a plurality of SDs **(1200A,1200B),** each intermediate SD **(1200B)** of said plurality of SDs **(1200A,1200B)** has controllable and non-invasive movement relative to said next outer one of said plurality of SDs **(1200A)** and the PIR, said movement selected from a group consisting of rotation, forward-backward movement, decentralization, tilt, angulation and any combination thereof.

At least one SD can be, as shown by the inner SD **(1200B),** a cylindrical ring. As shown, the cylindrical SD **(1200B)** will be almost fully or entirely inside the profile of the PIR **(1100)** or the next outer SD **(1200A).**

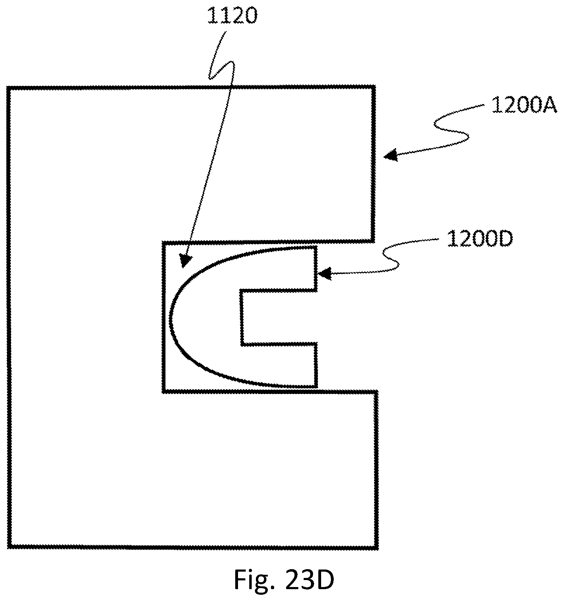

In the embodiment of **Fig. 23D****,** the inner SD **(1200D)** does not mate to an adjacent SD or the PIR via a male-female mating, such as disclosed above. In the embodiment of **Fig. 23D****,** the inner SD **(1200D)** sits in the holding groove **(1120)** of the outer SD or PIR **(1200A).** The outer side of the inner SD **(1200D),** the side in contact with the outer SD or PIR **(1200A),** has an oval profile. The outer side of the inner SD **(1200D)** can have any cross-sectional shape, rectangular, oval, circular, elliptical, polygonal and any combination thereof. The inner side of the inner SD **(1200A)** can have any cross-sectional shape, rectangular, oval, circular, elliptical, polygonal and any combination thereof. The two shapes need not match.

In the embodiment shown, the inner SD **(1200D)** is entirely within the profile of the outer SD or PIR **(1200A).** In other embodiments, the inner SD **(1200D)** extends beyond the profile of the outer SD or PIR **(1200A).**

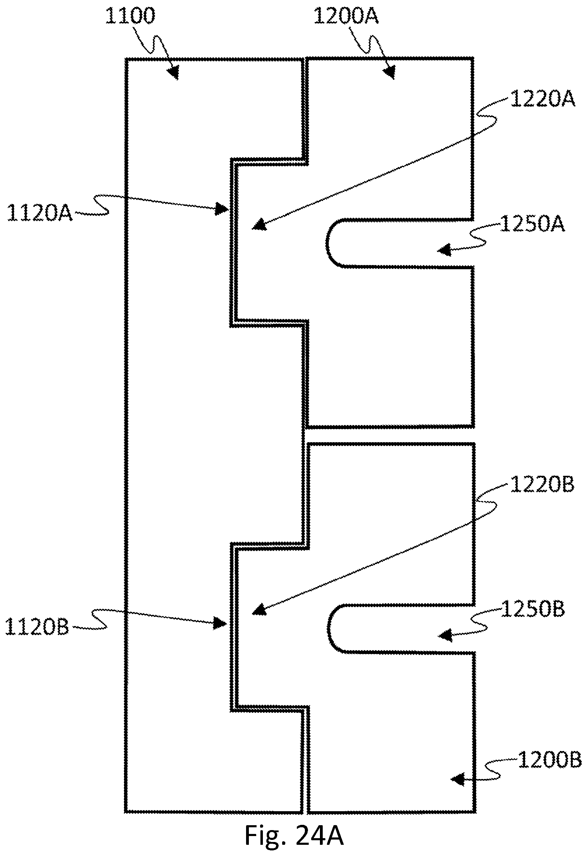

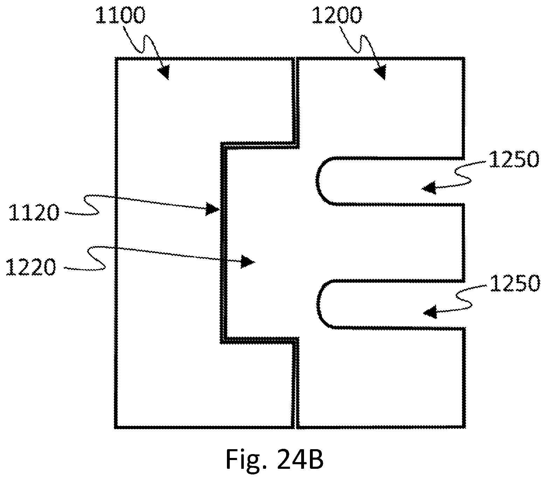

**Fig. 24A-B** schematically illustrates cross-sections of embodiments of a PIR **(1100)** configured to hold two SDs. **Fig. 24A** shows an embodiment with two SDs and **Fig 24B** shows an embodiment with one SD.

**Fig. 24A** schematically illustrates a cross-section of an exemplary embodiment of a PIR **(1100)** configured to hold two SDs **(1200A, 1200B),** one anteriorly in the eye **(1200A)** and one posteriorly **(1200B).** In this schematic illustration the PIR **(1100)** has two holding grooves **(1120A, 1120B).** The upper (outward from the eye) holding groove **(1120A)** snugly and rotatably holds the tongue **(1220A)** of the upper (outward from the eye) SD **(1200A),** while the lower (inward toward the eye) holding groove **(1120B)** snugly and rotatably holds the tongue **(1220B)** of the lower (inward toward the eye) SD **(1200B).** Each SD **(1200A, 1200B)** comprises a mounting groove **(1250A, 1250B)** configured by means of size and shape to hold an IOL **(2000,** not shown). It should be noted that in other embodiments, the holding grooves **(1120A, 1120B)** and tongues **(1220A, 1220A)** can be any of the SD-SD or SD-PIR connections disclosed herein and need not be the same; the mounting grooves **(1250A, 1250B)** can be any of the SD-haptic connections disclosed herein and need not be the same.

**Fig. 24B** schematically illustrates a cross-section of an embodiment of an SD **(1200)** comprising two grooves **(1250).** The PIR **(1100)** and the tongue **(1220)** and holding groove **(1120)** are shown for clarity. It should be noted that, in other embodiments, the holding groove **(1120)** and tongue **(1220)** can be any of the SD-haptic connections disclosed herein.

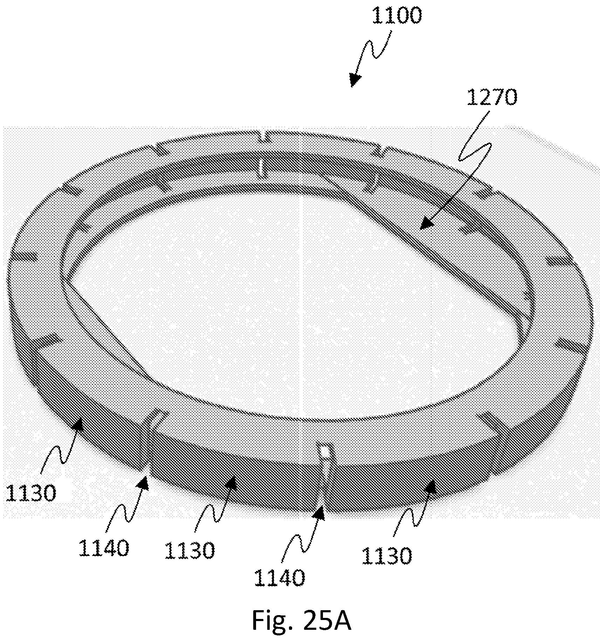

**Fig. 25A-B** illustrate embodiments of a PIR **(1100)** with segments **(1130A-C)** at least partially separated by interstices **(1140).** Each interstice **(1140)** links two segments **(1130).** The interstice(s) are configured to increase at least one of the compressibility and the bendability of the PIR, thereby making it easier to inset the PIR into an eye.

Both embodiments comprise IOL guides **(1270).** Other embodiments of a PIR **(1100)** with interstices **(1140A-C)** can have more or fewer IOL guides **(1270);** some embodiments have no IOL guides **(1270).**

The embodiments illustrated in **Fig. 25A-B** have 12 segments **(1130)** and 12 interstices **(1140).** The number of segments can be in a range from 1 to 40. In some embodiments, the PIR can also comprise a gap **(1110,** see **Fig. 6****).**

In the embodiment of **Fig. 25A****,** the interstices **(1140)** are cut into the outer perimeter of the PIR **(1100);** the segments **(1130)** are separate in the portion of the PIR **(1100)** configured to contact an eye and the segments **(1130)** are linked on their inner perimeter, the portion configured to contact an SD **(1200,** see, e.g., **Figs. 5-10****,** and **13-22).** For non-limiting example, for a U-shaped PIR **(1100),** as shown, the interstices **(1140)** are cut into the base of the U.

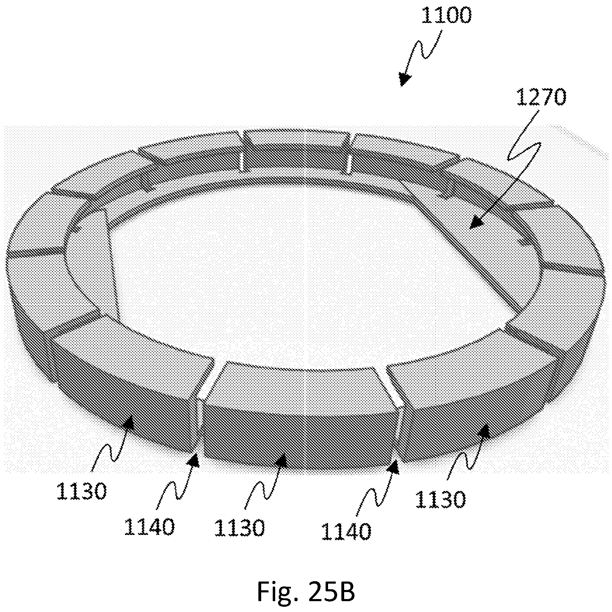

In the embodiment of **Fig. 25B****,** the interstices **(1140)** are cut into the inner perimeter of the PIR **(1100);** the segments **(1130)** are separate in the portion configured to contact an SD **(1200,** see, e.g., **Figs. 5-10****,** and **13-22)** and the segments are linked on their outer perimeter, the portion of the PIR **(1100)** configured to contact an eye. For non-limiting example, for a U-shaped PIR **(1100),** as shown, the interstices **(1140)** are cut into the arms of the U.

In some embodiments, the interstices **(1140)** are cut into only one side of an inner perimeter, for non-limiting example, into one arm of a U-shaped PIR **(1100).**

In some embodiments, the interstices **(1140)** are cut into one side of an inner perimeter and into the outer perimeter, non-limiting example, into one arm and the base of a U-shaped PIR **(1100).** Other variants will be obvious to one skilled in the art.

In some embodiments, two adjacent segments **(1130)** are linked along at least part of their mating edges by a more resilient material. The adjacent segments **(1130)** can have a linking portion of the segment material, can have a linking portion of a more resilient material, can have an interstice, and any combination thereof. The more resilient material can be softer than the PIR material, more flexible than the PIR material, more elastic than the PIR material, thinner than the PIR material, and any combination thereof.

It should be noted that some embodiments of the SD **(1200,** not shown) can comprise one or more segments **(1130).** For each pair of adjacent segments **(1130),** a gap **(1210,** see **Fig. 6****)** can separate the segments **(1130)** or the pair of adjacent segments **(1130)** can be linked by an interstice **(1140),** a more resilient material and any combination thereof, in a manner similar to the gaps **(1210,** see **Fig. 6****),** interstices **(1140)** and more resilient materials disclosed above. As disclosed above for the PIR, the interstice(s) are configured to increase at least one of the compressibility and the bendability of the PIR, thereby making it easier to inset the PIR into an eye.

In some embodiments, the SD is connected to the IOL but is not connected to the PIR. In these embodiments, the PIR protects the capsular bag from friction with the haptics, and the SD is connected directly to the lens and provides the torque to rotate it or the force to move it.

In some embodiments, the SD is non-circular, or is made out of several parts which support the haptics.

In some embodiments, the torque mechanism is connected directly to the haptics or optics of the IOL. In some variants of these embodiments, an SD can be connected to the haptics of the IOL without contact with the PIR.

In some embodiments, the torque mechanism is omitted and the rotation is done during microsurgery.

In some embodiments, rotation of the SD also induces axial movement, thereby enabling the effective refractive power of the lens (the number of diopters of correction) to be changed.

In some embodiments, the IOL is permanently attached to an SD. In some variants of these embodiments, the permanently attached SD is in contact with at least the distal end of the IOL haptic(s). In some variants of these embodiments, the permanently attached SD does not contact either the PIR or another SD, for non-limiting example, the distal end of the IOL haptic(s) extends outward beyond the outer edge of the permanently attached SD.

It should be noted that the IRS of the present invention enables rotation of conventional IOLs without the need for additional operations after implantation surgery to adjust the rotation. In addition, the IRS of the present invention is preferably designed to at least reduce and preferably prevent agglutination or fibrosis of the IOL to the capsular bag.

Furthermore, the IOL of the present invention allows easier and better centralization of lens inside the eye with respect to the eye's optical axis, which in turn leads to a more predictable lens position and, therefore, a better outcome for the patient.

An IRS with a solid back or a non-solid back can be used in case of a posterior capsular tear. For non-limiting example, if, either during or after an insertion procedure, the capsular bag-IRS-IOL complex becomes subluxated, repair of the subluxation by suturing the capsular bag-IRS-IOL complex to the sclera will be easier than repair of the subluxation by suturing a subluxated bag-IOL complex to the sclera.

The PIR can be rigid or flexible and any SD can be rigid or flexible. If a PIR or an SD is rigid, it must comprise a gap. The gap allows the diameter of the PIR to be reduced to enable insertion into the eye. In addition, a PIR or SD with a gap automatically adjusts itself to the size of the eye, so that only a single size is needed, or perhaps two, one for adults and one for children. A flexible PIR or SD can be folded for insertion and therefore needs no gap however, different sizes would be needed to fit different sized eyes. Typically, there will be 3 adult sizes and one size for children. The PIR and the SD(s) of the present invention each comprise at least one insertion aid portion, where the insertion aid portion enable sufficient reduction in at least one dimension of the PIR or SD so that the PIR or SD can be inserted into the eye. The insertion portion can comprise a flexible material, it can comprise a hinge, the PIR or SD can comprise a gap, and any combination thereof. The PIR or SD can be of flexible material, of rigid material and any combination thereof.

## Claims

1. An intraocular ring system (IOL) **(1000)** comprising:
a conventional intraocular lens (IOL) **(2000)** comprising an optic and at least two haptics **(2200),** at least one of the at least two haptics **(2200)** being flexible, the at least two haptics **(2200)** configured for holding the optic in position relative to an eye without subsidiary holding structures;
**characterized by** a support system for said IOL **(2000)** comprising
a primary intraocular ring (PIR) **(1100)** mountable to an eye; and
a secondary device (SD) **(1200)** configured for connection with said IOL **(2000),** said SD **(1200)** configured to be in mechanical communication with said PIR **(1100)** and configured for movement relative to at least one of said PIR **(1100)** and said IOL **(2000),** said movement selected from a group consisting of rotatable, forward-movable, backward-movable, decentralizable, tiltable, angulatable and any combination thereof;
wherein said connection of the SD **(1200)** to said IOL **(1000)** is via said at least two haptics **(2200);**
wherein said IOL **(2000)** has controllable and non-invasive movement relative to said PIR **(1100)** while said system is implanted in the eye, said movement of the IOL **(2000)** selected from a group consisting of rotation, forward-backward movement, decentralization, tilt, angulation and any combination thereof; and
further wherein said movement relative to said PIR **(1100)** is effectuated by a shaped ultrasonic field.

2. The intraocular ring system of claim 1, wherein said at least two haptics are selected from the group consisting of a plate haptic, a loop haptic or any combination thereof.

3. The intraocular ring system of claim 1, wherein said PIR is configured to be supported by a member of a group consisting of a zonula of the eye, a capsular bag of the eye and any combination thereof.

4. The intraocular ring system of claim 1, wherein at least one member of a group consisting of said PIR and SD has a back, said back is selected from a group consisting of a solid back, a non-solid back and a partial back.

5. The intraocular ring system of claim 4, wherein a non-solidity of said back is selected from a group consisting of a perforation, a slot, a hole, a regularly shaped gap, an irregularly shaped gap, a mesh, and any combination thereof.

6. The intraocular ring system of claim 4, wherein said back comprises transparent material.

7. The intraocular ring system of claim 1, wherein said SD has at least one IOL guide configured by means of size and shape to guide at least one of said at least two haptics into a correct location.

8. The intraocular ring system of claim 1, wherein said SD comprises at least one component support configured by means of size and shape to hold said IOL.

9. The intraocular ring system of claim 8, wherein said at least one component support is selected from a group consisting of a mounting groove, a V groove, a U, an indentation and any combination thereof.

10. The intraocular ring system of claim 9, wherein at least a portion of said at least two haptics is held snugly by said at least one component support.

11. The intraocular ring system of claim 1, wherein said SD is configured to move with respect to said PIR and wherein said movement is by means of controlled movement of said SD with respect to said PIR.

12. The intraocular ring system of claim 1, wherein said IOL is removably attached to said SD.

## Patentansprüche

1. Intraokulares Ringsystem (IOL) **(1000),** umfassend:
eine herkömmliche Intraokularlinse (IOL) **(2000),** die eine Optik und mindestens zwei Haptiken **(2200)** umfasst, wobei mindestens eine der mindestens zwei Haptiken **(2200)** flexibel ist und die mindestens zwei Haptiken **(2200)** darauf ausgelegt sind, die Optik ohne ergänzende Haltestrukturen relativ zu einem Auge in Position zu halten;
**gekennzeichnet durch** ein Trägersystem für die genannte IOL **(2000),** umfassend
einen primären intraokularen Ring (PIR) **(1100),** der an einem Auge angebracht werden kann; und
eine sekundäre Vorrichtung (SD) **(1200),** die auf die Verbindung mit der genannten IOL **(2000)** ausgelegt ist, wobei die genannte SD **(1200)** darauf ausgelegt ist, sich in mechanischer Verbindung mit dem genannten PIR **(1100)** zu befinden und auf eine Bewegung relativ zu mindestens dem genannten PIR **(1100)** bzw. der genannten IOL **(2000)** ausgelegt ist, wobei die genannte Bewegung aus einer Gruppe gewählt wird, die die Optionen drehbar, vorwärts beweglich, rückwärts beweglich, dezentralisierbar, kippbar, abwinkelbar und jede beliebige Kombination davon umfasst;
wobei die genannte Verbindung der SD **(1200)** mit der genannten IOL **(1000)** über die genannten mindestens zwei Haptiken **(2200)** erfolgt;
wobei die genannte IOL **(2000)** eine kontrollierbare und nicht-invasive Bewegung relativ zu dem genannten PIR **(1100)** vorsieht, während das genannte System in das Auge implantiert ist, und wobei die genannte Bewegung der IOL **(2000)** aus einer Gruppe gewählt wird, die aus Rotation, Vorwärts-Rückwärts-Bewegung, Dezentralisierung, Neigung, Abwinkelung und jeder beliebige Kombination davon besteht; und
wobei außerdem die genannte Bewegung relativ zu dem genannten PIR **(1100)** durch ein geformtes Ultraschallfeld bewirkt wird.

2. Intraokulares Ringsystem nach Anspruch 1, wobei die genannten mindestens zwei Haptiken aus der Gruppe gewählt werden, die aus einer Plattenhaptik, einer Schlaufenhaptik oder jeder beliebigen Kombination davon besteht.

3. Intraokulares Ringsystem nach Anspruch 1, wobei der genannte PIR darauf ausgelegt ist, von einem Element einer Gruppe gestützt zu werden, die aus einer Zonula des Auges, einem Kapselsack des Auges und jeder beliebigen Kombination davon besteht.

4. Intraokulares Ringsystem nach Anspruch 1, wobei mindestens ein Element einer Gruppe, die aus dem genannten PIR und der genannten SD besteht, eine Rückseite aufweist, wobei die genannte Rückseite aus einer Gruppe gewählt wird, die aus einer festen Rückseite, einer nicht-festen Rückseite und einer partiellen Rückseite besteht.

5. Intraokulares Ringsystem nach Anspruch 4, wobei eine Nicht-Festigkeit der genannten Rückseite aus einer Gruppe gewählt wird, die aus einer Perforation, einem Schlitz, einer Öffnung, einem regelmäßig geformten Spalt, einem unregelmäßig geformten Spalt, einem Netz und jeder beliebigen Kombination davon besteht.

6. Intraokulares Ringsystem nach Anspruch 4, wobei die genannte Rückseite transparentes Material umfasst.

7. Intraokulares Ringsystem nach Anspruch 1, wobei die genannte SD mindestens eine IOL-Führung aufweist, die durch Größe und Form darauf ausgelegt ist, mindestens eine der genannten mindestens zwei Haptiken in eine korrekte Lage zu bringen.

8. Intraokulares Ringsystem nach Anspruch 1, wobei die genannte SD mindestens eine Komponentenhalterung umfasst, die durch Größe und Form darauf ausgelegt ist, die IOL zu halten.

9. Intraokulares Ringsystem nach Anspruch 8, wobei die genannte mindestens eine Komponentenhalterung aus einer Gruppe gewählt wird, die aus einer Montagenut, einer V-Nut, einem U, einer Vertiefung und jeder beliebigen Kombination davon besteht.

10. Intraokulares Ringsystem nach Anspruch 9, wobei mindestens ein Teil der genannten mindestens zwei Haptiken von der mindestens einen Komponentenhalterung festgehalten wird.

11. Intraokulares Ringsystem nach Anspruch 1, wobei die genannte SD darauf ausgelegt ist, sich im Verhältnis zu dem genannten PIR zu bewegen und wobei die Bewegung mittels einer kontrollierten Bewegung der genannten SD im Verhältnis zu dem genannten PIR erfolgt.

12. Intraokulares Ringsystem nach Anspruch 1, wobei die genannte IOL abnehmbar an der genannten SD befestigt ist.

## Revendications

1. Système d'anneau intraoculaire (IOL) **(1000)** comprenant :
une lentille intraoculaire (IOL) **(2000)** conventionnelle comprenant une optique et au moins deux haptiques **(2200),** au moins une des au moins deux haptiques **(2200)** étant flexible, les au moins deux haptiques **(2200)** étant configurées pour maintenir l'optique en position par rapport à un œil sans structures de maintien subsidiaires ;
**caractérisé par** un système de support pour ladite IOL **(2000)** comprenant
un anneau intraoculaire primaire (PIR) **(1100)** pouvant être monté sur un œil ; et
un dispositif secondaire (SD) **(1200)** configuré pour une connexion avec ladite IOL **(2000),** ledit SD **(1200)** étant configuré pour être en communication mécanique avec ledit PIR **(1100)** et étant configuré pour le mouvement relatif par rapport à au moins l'un/l'une entre ledit PIR **(1100)** et ladite IOL **(2000),** ledit mouvement étant sélectionné dans un groupe constitué de mouvements rotatif, vers l'avant, vers l'arrière, décentralisable, inclinable, d'angulation et toute combinaison de ceux-ci ;
où ladite connexion du SD **(1200)** avec ladite IOL **(1000)** se fait par l'intermédiaire desdites au moins deux haptiques **(2200)** ;
où ladite IOL **(2000)** a un mouvement régulable et non invasif par rapport audit PIR **(1100)** pendant que ledit système est implanté dans l'œil, ledit mouvement de la IOL **(2000)** étant sélectionné dans un groupe constitué d'une rotation, d'un mouvement vers l'avant et vers l'arrière, d'une décentralisation, d'une inclinaison, d'une angulation et de toute combinaison de ceux-ci ; et
où, en outre, ledit mouvement par rapport audit PIR **(1100)** est effectué par un champ ultrasonique modelé.

2. Système d'anneau intraoculaire selon la revendication 1, où lesdites au moins deux haptiques sont sélectionnées dans le groupe constitué d'une haptique de plaque, d'une haptique en boucle ou d'une combinaison de celles-ci.

3. Système d'anneau intraoculaire selon la revendication 1, où ledit PIR est configuré pour être soutenu par un élément du groupe constitué d'une zonule de l'œil, d'un sac capsulaire de l'œil et de toute combinaison de ceux-ci.

4. Système d'anneau intraoculaire selon la revendication 1, où au moins un élément d'un groupe constitué desdits PIR et SD a un dos, ce dos étant sélectionné dans un groupe constitué d'un dos solide, un dos non solide et un dos partiel.

5. Système d'anneau intraoculaire selon la revendication 4, où la non-solidité dudit dos est sélectionnée dans un groupe constitué d'une perforation, d'une fente, d'un trou, d'un espace de forme régulière, d'un espace de forme irrégulière, d'une maille, et de toute combinaison de ceux-ci.

6. Système d'anneau intraoculaire selon la revendication 4, où ledit dos comprend un matériau transparent.

7. Système d'anneau intraoculaire selon la revendication 1, où ledit SD comporte au moins un guide de IOL configuré en fonction de la taille et de la forme pour guider au moins l'une desdites au moins deux haptiques dans un emplacement correct.

8. Système d'anneau intraoculaire selon la revendication 1, où ledit SD comprend au moins un support de composant configuré en fonction de la taille et de la forme pour maintenir ladite IOL.

9. Système d'anneau intraoculaire selon la revendication 8, où ledit au moins un support de composant est sélectionné dans un groupe constitué d'une rainure de montage, d'une rainure en V, en U, d'une indentation et de toute combinaison de celles-ci.

10. Système d'anneau intraoculaire selon la revendication 9, où au moins une partie desdites au moins deux haptiques est bien maintenue par ledit au moins un support de composant.

11. Système d'anneau intraoculaire selon la revendication 1, où ledit SD est configuré pour se déplacer par rapport audit PIR, et où ledit mouvement se fait au moyen d'un mouvement régulé dudit SD par rapport audit PIR.

12. Système d'anneau intraoculaire selon la revendication 1, où ladite IOL est fixée de manière amovible audit SD.
